# EUROPÄISCHE PATENTSCHRIFT

(11) **EP 4 178 456 B1**
(45) Veröffentlichungstag und Bekanntmachung des Hinweises auf die Patenterteilung: **14.02.2024**
(21) Anmeldenummer: 21745928.8
(22) Anmeldetag: 05.07.2021
(51) Int. Cl.: A61B 17/02, A61F 2/38, A61F 2/46, A61F 2/30

(54) **TIBIALES PROBEIMPLANTAT MIT GETRIEBE**
TIBIAL TRIAL IMPLANT WITH GEARS
IMPLANT D'ESSAI TIBIAL AVEC ENGRENAGES

(30) Priorität: 07.07.2020 DE 102020208501
(43) Veröffentlichungstag der Anmeldung: 17.05.2023
(73) Patentinhaber: Aesculap AG, 78532 Tuttlingen (DE)
(72) Erfinder: NONNENMANN, Martin, 78573 Wurmlingen (DE); MADER-ILG, Jennifer, 78606 Seitingen-Oberflacht (DE); ZOUAGHI, Housseyn, 52000 Chaumont (FR)
(74) Vertreter: Patentanwälte Ruff, Wilhelm, Beier, Dauster & Partner mbB
(86) Internationale Anmeldenummer: PCT/EP2021/068515
(87) Internationale Veröffentlichungsnummer: WO 2022/008444

(56) Entgegenhaltungen:
- WO-A1-2016/065396
- JP-A- 2015 177 970
- US-A- 5 733 292
- US-A1- 2012 158 152
- US-A1- 2013 138 112

## Beschreibung

Die Erfindung betrifft ein tibiales Probeimplantat gemäß dem Oberbegriff des Anspruchs 1.

Ein derartiges tibiales Probeimplantat ist sowohl aus der US 2013/138112 A1 als auch aus der JP 2015 177970 A bekannt, wobei jeweils ein über Drehelemente höhenverstellbares tibiales Probeimplantat offenbart wird. Ein weiteres höhenverstellbares tibiales Probeimplantat ist aus der US 2010/0063595 A1 bekannt und weist eine untere Platte und eine obere Platte auf. An einer Oberseite der oberen Platte ist eine Gleitfläche vorgesehen, die zum gleitenden Zusammenwirken mit einer femoralen Komponente vorgesehen ist. Die Unterseite der unteren Platte ist zur tibialen Fixierung vorgesehen. Weiter weist das tibiale Probeimplantat einen zwischen der oberen Platte und der unteren Platte angeordneten und mit beiden Platten wirkverbundenen Höheneinstellmechanismus auf. Der Höheneinstellmechanismus gestattet eine Einstellung der Gesamthöhe des Probeimplantats. Hierfür werden die obere Platte und die untere Platte mittels des Höheneinstellmechanismus relativ zueinander in Hochrichtung verlagert. Bei dem bekannten tibialen Probeimplantat weist der Höheneinstellmechanismus eine Scherengelenkanordnung auf, die einends fest mit der unteren Platte und andernends fest mit der oberen Platte verbunden ist. Zur manuellen Betätigung des Höheneinstellmechanismus ist ein an der unteren Platte schwenkbeweglich festgelegter Schwenkhebel vorgesehen.

Aufgabe der Erfindung ist es, ein tibiales Probeimplantat der eingangs genannten Art bereitzustellen, das einen gegenüber dem Stand der Technik verbesserten Aufbau aufweist und Vorteile bei der Anwendung bietet.

Diese Aufgabe wird durch das Bereitstellen eines tibialen Probeimplantats mit den Merkmalen des Anspruchs 1 gelöst. Das erfindungsgemäß vorgesehene Kurvengetriebe gestattet eine besonders vorteilhafte Einstellung der Höhe der Gleitfläche und einen besonders vorteilhaften Aufbau des tibialen Probeimplantats. Denn die antriebsseitige Drehbewegung des Antriebsrads ist über das Zusammenwirken der Steuerkurve mit dem Stützabschnitt auf einfache und robuste Weise in eine abtriebsseitige Bewegung des Oberteils und der daran angeordneten Gleitfläche übertragbar. Durch eine entsprechende Gestaltung der Steuerkurve sind mit geringem konstruktivem Aufwand unterschiedlichste proportionale oder nicht proportionale Übersetzungsverhältnisse zwischen der antriebsseitigen und der abtriebsseitigen Bewegung darstellbar. Zudem kann insbesondere aufgrund der parallel zur Hochrichtung orientierten Drehachse des Antriebsrads eine in Hochrichtung besonders kompakte Bauweise des tibialen Probeimplantats erreicht werden. Im Ergebnis wird durch die erfindungsgemäße Lösung ein einfacher, bauraumsparender und gleichzeitig robuster Aufbau erreicht. Das Antriebsrad ist zur wenigstens mittelbaren manuellen und/oder werkzeuggetriebenen Betätigung durch medizinisches Personal eingerichtet. Beispielsweise kann das Antriebsrad an seinem Außenumfang einen hierfür vorgesehenen Betätigungsabschnitt aufweisen. Alternativ oder zusätzlich kann ein zu einer manuellen und/oder werkzeuggetriebenen Betätigung eingerichtetes Betätigungselement vorgesehen sein, das dem Antriebsrad vorgeschaltet ist und mit diesem zusammenwirkt, so dass eine Bewegung des Betätigungselements auf das Antriebsrad übertragbar ist. Das Antriebsrad ist vorzugsweise gleitend an dem Unterteil gelagert. Die Steuerkurve wirkt bei einer Drehung des Antriebsrads gleitend mit dem Stützabschnitt des Abtriebselements zusammen. Der Stützabschnitt ist in Hochrichtung auf der Steuerkurve abgestützt. Bei einer Drehung des Antriebsrads findet eine relative Gleitbewegung zwischen der Steuerkurve und dem Stützabschnitt statt. Mit anderen Worten ausgedrückt, gleitet die Steuerkurve unter dem Stützabschnitt hindurch. Der Stützabschnitt wird hierdurch aufgrund der Steigung der Steuerkurve in Hochrichtung nach oben gedrückt. Die Steuerkurve und der Stützabschnitt können punkt-, linienförmig und/oder flächig zusammenwirken. Die Steuerkurve kann eine konstante oder veränderliche Steigung aufweisen. Das Abtriebselement ist fest mit dem Oberteil verbunden. Vorzugsweise ist das Abtriebselement einstückig an das Oberteil angeformt. Vorzugsweise ist das Abtriebselement unterseitig an dem Oberteil angeordnet. Die Gleitfläche ist vorzugsweise an dem Oberteil ausgebildet und bildet dessen Oberseite. Alternativ kann die Gleitfläche an einem separaten Teil ausgebildet sein, das mit der Oberseite des Oberteils verbunden ist. Das Unterteil ist zur tibialen Fixierung vorgesehen. In der Anwendung kann die Unterseite des Unterteils unmittelbar am proximalen Ende der Tibia fixiert sein. Alternativ kann das Unterteil an einem unmittelbar tibial befestigten Tibialprobeplateau oder dergleichen fixiert sein. Das Unterteil und das Oberteil können jeweils ein- oder mehrteilig gestaltet sein. Vorzugsweise weisen das Unterteil und das Oberteil jeweils eine plattenförmige Grundform auf, so dass auch von einer unteren bzw. oberen Platte gesprochen werden kann. In der ersten Einstellposition weist das tibiale Probeimplantat eine in Hochrichtung erstreckte Gesamtbauhöhe auf, die kleiner ist als eine Gesamtbauhöhe in der zweiten Einstellposition. Dementsprechend ist die Gleitfläche in der ersten Einstellposition in Hochrichtung näher am Unterteil positioniert als in der zweiten Einstellposition.

In Ausgestaltung der Erfindung ist die Steuerkurve durch eine mit einer konstanten Steigung wendelförmig koaxial um die erste Drehachse erstreckte Steuerfläche gebildet. Die Steuerkurve beschreibt somit eine in Hochrichtung ansteigende Schraubenlinie. Durch die konstante Steigung kann auf konstruktiv einfache Weise ein lineares Bewegungsgesetz zwischen dem Antriebsrad und dem Abtriebselement und somit eine proportionale Bewegungs- und Kraftübertragung erreicht werden. Dies im Unterschied zu aus dem Stand der Technik bekannten Scherengelenkanordnungen zur Höheneinstellung der Gleitfläche. Diese Ausgestaltung der Erfindung ermöglicht somit weitere Vorteile bei der Anwendung. Zusätzlich kann weiterer Bauraum eingespart werden.

In weiterer Ausgestaltung der Erfindung ist der Stützabschnitt durch eine mit einer konstanten Steigung wendelförmig koaxial um die erste Drehachse erstreckte Stützfläche gebildet. Durch die flächige Ausgestaltung des Stützabschnitts kann insbesondere eine verringerte Flächenpressung an der Steuerkurve erreicht werden. Hierdurch kann der Höheneinstellmechanismus leichtgängig und verschleißarm betrieben werden.

In weiterer Ausgestaltung der Erfindung sind das Oberteil und das Unterteil mittels einer zwischen dem wenigstens einen Antriebsrad und dem wenigstens einen Abtriebselement ausgebildeten Steckverbindung entlang einer Steckachse relativ zueinander gleitbeweglich lösbar zusammengesteckt, wobei die Steckachse koaxial zu der ersten Drehachse orientiert ist. Dies ist eine besonders vorteilhafte Ausgestaltung der Erfindung. Dem Kurvengetriebe kommt hierbei eine besonders vorteilhafte Mehrfachfunktion zu. Denn zusätzlich zu der Einstellung der Höhe der Gleitfläche fungiert der Höheneinstellmechanismus als lösbare Schnittstelle zwischen dem Ober- und dem Unterteil. Zu diesem Zweck ist die Steckverbindung zwischen dem Antriebsrad und dem Abtriebselement ausgebildet. Die Steckverbindung ist derart eingerichtet, dass das Abtriebselement - und damit auch das fest mit dem Abtriebselement verbundene Oberteil - nach oben von dem Unterteil abgezogen werden können. Zu diesem Zweck ist die Steckachse koaxial zu der ersten Drehachse und damit parallel zur Hochrichtung orientiert. Diese Ausgestaltung der Erfindung ermöglicht, dass das Unterteil und die daran gelagerten Komponenten des Höheneinstellmechanismus auf einfache Weise mit unterschiedlichen Oberteilen, die beispielsweise unterschiedlich ausgestaltete Gleitflächen aufweisen, verbunden werden kann. Dies im Unterschied zu aus dem Stand der Technik bekannten Lösungen, die eine unlösbare oder wenigstens manuell nicht lösbare Kopplung zwischen dem Unterteil und dem Oberteil vorsehen. Die unterschiedlichen Oberteile können sich beispielsweise auch in der anterior-posterioren Dimension, der medial-lateralen Dimension, der Höhe und/oder bei einer asymmetrischen Ausgestaltung der Gleitfläche auch durch spiegelsymmetrische Varianten für das linke, beziehungsweise das rechte Knie unterscheiden.

In weiterer Ausgestaltung der Erfindung weist das wenigstens eine Antriebsrad eine koaxial zu der ersten Drehachse erstreckte Zylinderbohrung auf, in welche ein komplementärer Steckzylinder des wenigstens einen Abtriebselements unter Ausbildung der lösbaren Steckverbindung eingesteckt ist. Der Steckzylinder ist entlang der Steckachse und damit entlang der Hochrichtung gleitbeweglich in die Zylinderbohrung eingesteckt. Dies ermöglicht zum einen ein einfaches Lösen der Steckverbindung. Zum anderen ermöglicht dies die zur Einstellung der Höhe der Gleitfläche erforderliche Relativbewegung zwischen dem Ober- und dem Unterteil.

In weiterer Ausgestaltung der Erfindung weist der Höheneinstellmechanismus ein dem Kurvengetriebe vorgeschaltetes Schneckengetriebe auf. "Vorgeschaltet" meint, dass das Schneckengetriebe dem Kurvengetriebe in Antriebsrichtung vorgelagert ist. Mit anderen Worten ausgedrückt, ist das Kurvengetriebe mittels des Schneckengetriebes angetrieben. Das vorgeschaltete Schneckengetriebe ermöglicht auf konstruktiv einfache und bauraumsparende Weise besonders hohe Übersetzungsverhältnisse. Hierdurch kann eine präzise Einstellung der Höhe der Gleitfläche erreicht werden. Zudem können erforderlichenfalls hohe Spreizkräfte zwischen dem Unter- und dem Oberteil erzeugt werden.

In weiterer Ausgestaltung der Erfindung weist das Schneckengetriebe eine Selbsthemmung auf. "Selbsthemmung" meint, dass das Schneckengetriebe lediglich über seinen Getriebeeingang antreibbar ist. Umgekehrt ist ein Antrieb des Schneckengetriebes ausgehend von seinem Getriebeausgang, beispielsweise reibungs- und/oder übersetzungsbedingt, nicht möglich. Hierdurch kann auf eine Verriegelung des Oberteils in den unterschiedlichen Einstellpositionen verzichtet werden. Dies ermöglicht eine diesbezüglich weiter vereinfachte Konstruktion. Zudem kann auf eine für diesen Zweck selbsthemmende Auslegung des Kurvengetriebes verzichtet werden. Das Kurvengetriebe kann somit vorteilhaft in erster Linie im Hinblick auf ein zu erzielendes Bewegungsgesetz zwischen dem Unterteil und dem Oberteil ausgelegt werden.

In weiterer Ausgestaltung der Erfindung weist das Schneckengetriebe eine Schneckenantriebswelle auf, die um eine senkrecht zur Hochrichtung orientierte zweite Drehachse drehbar an dem Unterteil gelagert ist, wobei die Schneckenantriebswelle mit einem verzahnten Außenumfangsabschnitt des wenigstens einen Antriebsrads oder mit einem dem Antriebsrad vorgeschalteten Schneckenrad zusammenwirkt. Durch die entsprechende Orientierung der zweiten Drehachse kann eine in Hochrichtung kompakte Bauweise des tibialen Probeimplantats erreicht werden. Die Schneckenantriebswelle kann unmittelbar oder mittelbar auf das wenigstens eine Antriebsrad wirken. Zu diesem Zweck kann das wenigstens eine Antriebsrad an seinem Außenumfang einen verzahnten Abschnitt, nämlich den verzahnten Außenumfangsabschnitt, aufweisen. Die Verzahnung der Schneckenantriebswelle kämmt zum Antrieb des Antriebsrads in der Verzahnung des Außenumfangsabschnitts. Sofern ein lediglich mittelbarer Antrieb vorgesehen ist, wirkt die Schneckenantriebswelle auf das dem Antriebsrad vorgeschaltete Schneckenrad. Letzteres ist vorzugsweise um eine dritte Drehachse drehbar an dem Unterteil gelagert, die parallel zur Hochrichtung orientiert ist.

In weiterer Ausgestaltung der Erfindung weist die Schneckenantriebswelle einends einen Drehbetätigungsabschnitt auf, der zur manuellen und/oder werkzeuggetriebenen Drehbetätigung der Schneckenantriebswelle um die zweite Drehachse eingerichtet ist. Der Drehbetätigungsabschnitt kann insbesondere als Drehknopf, Kurbel oder dergleichen zur manuellen Betätigung gestaltet sein. Alternativ oder zusätzlich kann einends des Drehbetätigungsabschnitts eine Werkzeugfläche zur werkzeuggetriebenen Drehbetätigung vorgesehen sein. Der Drehbetätigungsabschnitt ist von außen zugänglich an dem Unterteil angeordnet.

In weiterer Ausgestaltung der Erfindung ist das wenigstens eine Antriebsrad und/oder die Schneckenantriebswelle an einer Gleitlagerfläche des Unterteils gleitend drehbar gelagert, wobei die Gleitlagerfläche mehrere radiale Spülausbuchtungen aufweist. Die Spülausbuchtungen ermöglichen ein einfaches Durchspülen der zwischen dem Antriebsrad und/oder der Schneckenantriebswelle und dem Unterteil ausgebildeten Gleitlagerung. Hierdurch kann eine besonders hygienische Anwendung des tibialen Probeimplantats erreicht werden.

Weiter gemäß der Erfindung ist zwischen dem Unterteil und dem wenigstens einen Antriebsrad eine Skalenanzeige ausgebildet, die ein Ableseelement und eine Skale aufweist und ein Ablesen der Einstellposition des Oberteils und/oder der Höhe der Gleitfläche gestattet. Bei einer Drehbewegung des Antriebsrads werden das Ableseelement und die Skale relativ zueinander bewegt. Das Ableseelement kann an dem Unterteil und die Skale kann an dem Antriebsrad angeordnet sein, oder umgekehrt. Bei einer weiteren Ausgestaltung sind mehrere Skalenanzeigen ausgebildet, um das Ablesen aus unterschiedlichen Blickwinkeln zu ermöglichen. Alternativ können bei zwei oder mehr Skalenanzeigen die abzulesenden Werte im Wechsel auf den einzelnen Skalen angeordnet sein, um trotz fein abgestufter Werte eine gute Lesbarkeit zu ermöglichen.

In weiterer Ausgestaltung der Erfindung ist das Unterteil zweischalig gestaltet und weist eine Oberschale und eine Unterschale auf, wobei das wenigstens eine Antriebsrad und/oder die Schneckenantriebswelle zwischen der Oberschale und der Unterschale gehalten sind. In einem miteinander zusammengefügten Zustand bilden die Oberschale und die Unterschale ein Gehäuse zur Lagerung des wenigstens einen Antriebsrads und/oder der Schneckenantriebswelle.

In weiterer Ausgestaltung der Erfindung ist der Höheneinstellmechanismus in Bezug auf eine vertikale Mittellängsebene wenigstens weitgehend, vorzugsweise vollständig, spiegelsymmetrisch gestaltet, wobei wenigstens zwei Antriebsräder und wenigstens zwei Abtriebselemente vorgesehen sind. Die Antriebsräder und die Abtriebselemente sind somit in Querrichtung nebeneinanderliegend angeordnet. Sofern dem Kurvengetriebe ein Schneckengetriebe vorgeschaltet ist, treibt dessen Schneckenantriebswelle vorzugsweise beide Antriebsräder an. Zu diesem Zweck ragt die Schneckenantriebswelle in Längsrichtung zwischen die beiden Antriebsräder.

In weiterer Ausgestaltung der Erfindung sind wenigstens zwei Antriebsräder und ein Steuerrad vorhanden, die jeweils eine Stirnverzahnung aufweisen, wobei das Steuerrad mit beiden Antriebsrädern stirnverzahnt ist. Die wenigstens zwei Antriebsräder sind vorzugsweise in Querrichtung voneinander beabstandet angeordnet. Das Steuerrad ist in Querrichtung zwischen den wenigstens zwei Antriebsrädern angeordnet und diesen vorgeschaltet. Das Steuerrad dient einer Kraft- und Bewegungskopplung der beiden Antriebsräder. Infolge der Kopplung mittels des Steuerrads sind die beiden Antriebsräder um ihre jeweilige Drehachse zueinander synchron drehbeweglich. Vorzugsweise ist das Steuerrad an dem Unterteil drehbeweglich gelagert. Bei einer Ausgestaltung ist das Steuerrad zur unmittelbaren manuellen Drehbetätigung eingerichtet. Bei einer weiteren Ausgestaltung kann das Steuerrad zum Zusammenwirken mit einer Betätigungsmechanik oder dergleichen eingerichtet sein. Bei den Stirnverzahnungen handelt es sich jeweils um eine Außenverzahnung. Die Drehachsen der wenigstens zwei Antriebsräder und des Steuerrads sind zueinander parallel orientiert.

In weiterer Ausgestaltung der Erfindung weist das wenigstens eine Steuerrad eine weitere Steuerkurve auf, die mit einem weiteren Stützabschnitt eines weiteren Abtriebselements zusammenwirkt. Das Steuerrad fungiert somit gleichsam als weiteres Antriebsrad. Mit anderen Worten ausgedrückt, sind bei dieser Ausgestaltung wenigstens drei Antriebsräder und drei Abtriebselemente vorhanden, wobei eines der Antriebsräder gleichsam durch das wenigstens eine Steuerrad gebildet ist. Dem wenigstens einen Steuerrad kommt hierdurch eine besonders vorteilhafte Mehrfachfunktion zu. Hierdurch wird ein besonders kompakter und gleichzeitig besonders robuster Aufbau erreicht.

Weiter gemäß der Erfindung ist das wenigstens eine Antriebsrad bewegungsübertragend mit wenigstens einem an dem Unterteil drehbeweglich gelagerten Anzeigerad gekoppelt, wobei das Anzeigerad eine Skale aufweist, die ein Ablesen der Einstellposition des Oberteils und/oder der Höhe der Gleitfläche gestattet. Die Bewegungsübertragung zwischen dem Antriebsrad und dem Anzeigerad kann kontinuierlich oder intermittierend sein. Zur Bewegungsübertragung kann das Anzeigerad mit dem Antriebsrad verzahnt oder auf sonstige Weise, beispielsweise mittels eines Mitnehmerelements, gekoppelt sein. Vorzugsweise ist das Anzeigerad an einem Außenumfang des Unterteils angeordnet und/oder relativ zu dem wenigstens einen Antriebsrad in radialer Richtung nach außen versetzt. Die Skale weist vorzugsweise eine Abfolge von Teilstrichen und/oder Ziffern auf. Vorzugsweise ist die Skale auf einem Außenumfang des Anzeigerads angeordnet.

In weiterer Ausgestaltung der Erfindung ist wenigstens ein drehfest mit dem wenigstens einen Antriebsrad verbundenes Mitnehmerelement vorhanden, das intermittierend mit dem Anzeigerad zusammenwirkt, wodurch eine kontinuierliche Drehbewegung des Antriebsrads eine schrittweise Drehbewegung des Anzeigerads bewirkt. Die auf diese Weise bewirkte schrittweise Drehbewegung des Anzeigerads führt zu einer schrittweisen Anzeige der jeweils erreichten Einstellpositionen des Oberteils. Zwischenpositionen werden dementgegen nicht angezeigt. Das Mitnehmerelement kann einstückig an dem Antriebsrad ausgebildet oder als separat gefertigtes und hiernach mit dem Antriebsrad verbundenes Bauteil ausgeführt sein. Das Mitnehmerelement wirkt intermittierend mit einem hierfür vorgesehenen Abschnitt des Anzeigerads zusammen.

In weiterer Ausgestaltung der Erfindung ist eine Rasteinrichtung vorhanden, die ein drehfest mit dem wenigstens einen Antriebsrad wirkverbundenes Rastelement und ein an dem Unterteil angeordnetes komplementäres Gegenrastelement aufweist, wobei das Rastelement und das Gegenrastelement in definierten Drehstellungen des wenigstens einen Antriebsrads unter Ausbildung einer überrastbaren Rastverbindung zusammenwirken. Die definierten Drehstellungen des wenigstens einen Antriebsrads korrespondieren mit vorgegebenen und/oder zu erreichenden Einstellpositionen des Oberteils. Hierdurch erhält ein Benutzer eine unmittelbare taktile und/oder akustische Rückmeldung, sobald die besagte Drehstellung und/oder Einstellposition erreicht ist. Diese Rückmeldung ergibt sich durch das Verrasten der Rasteinrichtung. Die Rastverbindung zwischen dem Rastelement und dem komplementären Gegenrastelement ist überrastbar, so dass eine weitergehende Drehbewegung des Antriebsrads auch nach Erreichen der definierten Drehstellung möglich ist. Dabei geht das Überrasten - also das Lösen der zuvor etablierten Rastverbindung - mit einer erneuten taktilen und/oder akustischen Rückmeldung für den Benutzer einher.

In weiterer Ausgestaltung der Erfindung ist ein mit dem Unterteil lösbar verbindbarer Handgriff mit einer Betätigungsmechanik vorhanden, wobei die Betätigungsmechanik ein Betätigungsrad aufweist, das in einem mit dem Unterteil verbundenen Zustand des Handgriffs mit dem wenigstens einen Antriebsrad wirkverbunden ist. Der Handgriff ermöglicht eine ergonomische Betätigung des Höheneinstellmechanismus. Zu diesem Zweck ist die Betätigungsmechanik des Handgriffs dem Höheneinstellmechanismus, genauer: dessen wenigstens einem Antriebsrad, vorgeschaltet. Eine manuelle Betätigung der Betätigungsmechanik bewirkt eine Drehbewegung des wenigstens einen Antriebsrads und somit eine entsprechende Veränderung der Einstellposition des Oberteils. Zudem erlaubt der Handgriff eine sichere und ergonomische Handhabung des Unterteils. Zu diesem Zweck ist der Handgriff lösbar mit dem Unterteil verbindbar. Vorzugsweise ist hierfür eine lösbare Rast-, Schnapp-, Klemm- und/oder Steckverbindung zwischen dem Handgriff und dem Unterteil vorhanden, die wenigstens ein dem Handgriff zugeordnetes Verbindungselement und ein hierzu komplementäres Gegenverbindungselement an dem Unterteil aufweist. Das Betätigungsrad der Betätigungsmechanik ist - in dem mit dem Unterteil verbundenen Zustand des Handgriffs - lösbar mit dem wenigstens einen Antriebsrad wirkverbunden. Vorzugsweise sind das Betätigungsrad und das wenigstens eine Antriebsrad zu diesem Zweck wenigstens mittelbar miteinander verzahnt. Hierfür weisen das Betätigungsrad und das wenigstens eine Antriebsrad vorzugsweise jeweils eine Stirnverzahnung auf. Ein Übersetzungsverhältnis zwischen der Drehbewegung des Betätigungsrads und der Drehbewegung des wenigstens einen Antriebsrads kann auf einfache Weise mittels einer entsprechenden Gestaltung der Betätigungsmechanik beeinflusst werden. Je nach gewähltem Übersetzungsverhältnis kann eine besonders präzise und leichtgängige oder eine besonders rasche Einstellung des Höheneinstellmechanismus erreicht werden.

In weiterer Ausgestaltung der Erfindung weist der Handgriff wenigstens ein an seinem distalen Ende angeordnetes Verbindungselement auf, das zur Verbindung des Handgriffs mit dem Unterteil lösbar mit einem komplementären Verbindungselement des Unterteils verbindbar ist. Diese Ausgestaltung erlaubt einen besonders einfachen und robusten Aufbau. Vorzugsweise sind die Verbindungselemente als Rast- bzw. Gegenrastelement gestaltet. Alternativ ist eine Steck- oder Klemmverbindung mit entsprechend gestalteten Verbindungselementen denkbar. Bei einem Etablieren und einem Lösen der Rastverbindung wird das Rastelement und/oder das komplementäre Gegenrastelement elastisch deformiert. Vorzugsweise sind wenigstens zwei in Querrichtung voneinander beabstandet angeordnete Rastelemente und komplementäre Gegenrastelemente vorhanden. Der Handgriff ist zwischen dem distalen Ende und seinem proximalen Ende längserstreckt. Vorzugsweise ist das Betätigungsrad des Betätigungsmechanismus distal angeordnet.

Weitere Vorteile und Merkmale der Erfindung ergeben sich aus der nachfolgenden Beschreibung bevorzugter Ausführungsbeispiele der Erfindung, die anhand der Zeichnungen dargestellt sind.
- Fig. 1: zeigt in perspektivischer Ansicht eine Ausführungsform eines erfindungsgemäßen tibialen Probeimplantats,
- Fig. 2: das tibiale Probeimplantat nach Fig. 1 in einer perspektivischen Explosionsansicht,
- Fig. 3: eine weitere perspektivische Explosionsansicht,
- Fig. 4: das tibiale Probeimplantat nach den Fig. 1 bis 3 in einer schematischen Schnittdarstellung entlang eines Schnittes A-A gemäß Fig. 5,
- Fig. 5: eine schematische Frontansicht des tibialen Probeimplantats nach den Fig. 1 bis 4,
- Fig. 6: eine weitere perspektivische Ansicht unter zeichnerischer Ausblendung einzelner Bauteile und/oder Abschnitte des tibialen Probeimplantats,
- Fig. 7: in perspektivischer Detailansicht zwei Antriebsräder eines Höheneinstellmechanismus des tibialen Probeimplantats nach den Fig. 1 bis 6,
- Fig. 8: eine weitere perspektivische Ansicht ähnlich der Darstellung nach Fig. 6 in einer schräg von unten nach oben gerichteten Blickrichtung,
- Fig. 9: eine der Fig. 8 entsprechende Ansicht unter zusätzlicher zeichnerischer Ausblendung der Antriebsräder,
- Fig. 10: in perspektivischer Ansicht eine weitere Ausführungsform eines erfindungsgemäßen tibialen Probeimplantats zusammen mit einem Tibia-Probeplateau und in einer ersten Einstellposition,
- Fig. 11: das tibiale Probeimplantat nach Fig. 10 in einer zweiten Einstellposition,
- Fig. 12: eine perspektivische Explosionsansicht des tibialen Probeimplantats nach den Fig. 10 und 11,
- Fig. 13: in perspektivischer Ansicht ein Oberteil des tibialen Probeimplantats nach den Fig. 10 bis 12 mit Blickrichtung auf eine Unterseite, an welcher Abtriebselemente eines Höheneinstellmechanismus angeordnet sind,
- Fig. 14: in perspektivischer Ansicht ein Unterteil des tibialen Probeimplantats nach den Fig. 10 bis 12,
- Fig. 15: eine weitere perspektivische Darstellung des Unterteils in einer teilweise explodierten Ansicht,
- Fig. 16: eine weitere Ansicht des Unterteils mit Blickrichtung auf einzelne Bauteile und/oder Abschnitte des Höheneinstellmechanismus,
- Fig. 17: in perspektivischer Ansicht ein Handgriff des tibialen Probeimplantats nach den Fig. 10 bis 12, der lösbar mit dessen Unterteil verbindbar ist und eine Betätigungsmechanik zur Betätigung des Höheneinstellmechanismus aufweist und
- Fig. 18: in perspektivischer Detaildarstellung ein distales Ende des Handgriffs nach Fig. 17.

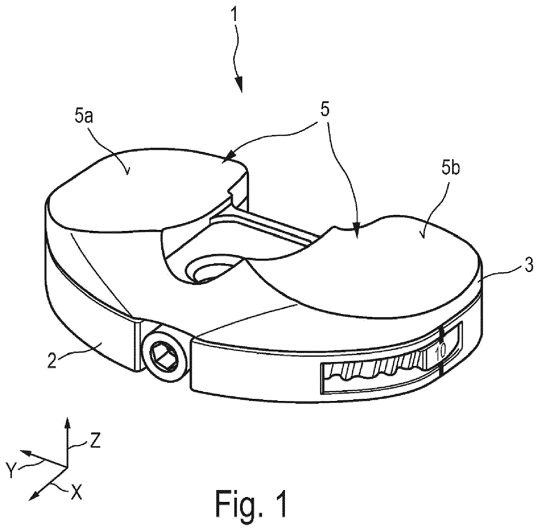

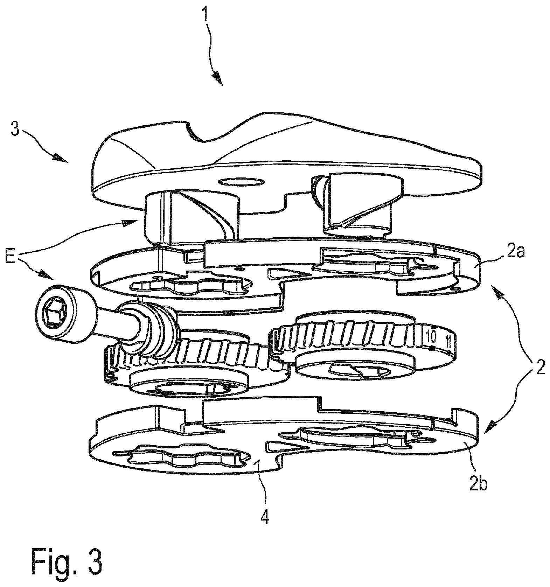

Gemäß den Fig. 1 bis 3 ist ein tibiales Probeimplantat 1 zur Verwendung bei einer Kniegelenkersatzoperation vorgesehen. Das tibiale Probeimplantat 1 wird in der medizinischen Fachsprache oftmals auch als "Probegleitfläche" bezeichnet.

Das tibiale Probeimplantat 1 weist ein Unterteil 2, ein Oberteil 3 und einen Höheneinstellmechanismus E auf.

Das Unterteil 2 ist zur tibialen Fixierung vorgesehen. Das heißt, bei einer Verwendung des tibialen Probeimplantats 1 ist das Unterteil 2 mittelbar oder unmittelbar an einem proximalen Ende einer Tibia angeordnet und festgelegt. Bei der gezeigten Ausführungsform ist eine mittelbare Fixierung des Unterteils 2 vorgesehen, bei welcher eine Unterseite 4 des Unterteils 2 mit einem sog. Tibia-Probeplateau zusammenwirkt, das mit dem proximalen Ende der Tibia verschraubt oder in dieses einzementiert sein kann.

Bei der gezeigten Ausführungsform weist das Unterteil 2 eine plattenförmige Grundform auf. Demgemäß ist das Unterteil 2 entlang einer Längsrichtung X und einer Querrichtung Y jeweils vergleichsweise weiter erstreckt als in einer Hochrichtung Z.

Vorliegend weist das Unterteil 2 eine zweischalige Gestaltung mit einer Oberschale 2a und einer Unterschale 2b auf, was auf noch näher beschriebene Weise vorteilhaft, aber nicht zwingend erforderlich ist. Die Unterschale 2b ist in Bezug auf die Hochrichtung Z unterhalb der Oberschale 2a angeordnet. Die Unterschale 2b weist die Unterseite 4 auf. In einem miteinander zusammengefügten Zustand bilden die Oberschale 2a und die Unterschale 2b einen nicht näher bezeichneten Aufnahmeraum für noch näher beschriebene Bauteile und/oder Abschnitte insbesondere des Höheneinstellmechanismus E aus. Im betriebsfertig montierten Zustand (Fig. 1) sind die Oberschale 2a und die Unterschale 2b in einer dem Fachmann bekannten Weise kraft-, form- und/oder stoffschlüssig miteinander zusammengefügt. Beispielsweise können die Oberschale 2a und die Unterschale 2b miteinander verschraubt, verklebt, verschweißt und/oder verrastet sein.

Das Oberteil 3 ist in Hochrichtung Z oberhalb des Unterteils 2 angeordnet und weist eine oberseitig angeordnete Gleitfläche 5 auf. Die Gleitfläche 5 ist bei der gezeigten Ausführungsform durch zwei Teilgleitflächen 5a, 5b gebildet, die in Querrichtung Y durch eine nicht näher bezeichnete oberseitige Ausnehmung an dem Oberteil 3 voneinander beabstandet sind. Die Gleitfläche 5 ist zum gleitenden Zusammenwirken mit einer femoralen Komponente vorgesehen. Diese femorale Komponente kann durch ein distales Ende eines Femurs oder ein an diesem fixiertes femorales Probeimplantat mit einer entsprechend gestalteten Gleitfläche gebildet sein. Die anhand der Figuren ersichtliche Formgebung der Gleitfläche 5 ist in einer dem Fachmann bekannten Weise der tibialen Gelenkfläche eines Femorotibialgelenkes nachempfunden.

Die Gleitfläche 5 ist bei der gezeigten Ausführungsform unmittelbar an dem Oberteil 3 ausgebildet und bildet insoweit dessen Oberseite. Eine solche Gestaltung ist vorteilhaft, aber im Hinblick auf die vorliegende Erfindung als nicht zwingend zu erachten. Bei einer nicht gezeigten Ausführungsform ist die Gleitfläche an einer separat von dem Oberteil gefertigten Komponente angeordnet, die in einem verwendungsbereiten Zustand fest mit dem Oberteil verbunden ist.

Der Höheneinstellmechanismus E dient einer Einstellung einer in Hochrichtung Z erstreckten Gesamtbauhöhe des tibialen Probeimplantats 1. Mit anderen Worten ausgedrückt, kann das Oberteil 3 und damit die Gleitfläche 5 mittels des Höheneinstellmechanismus E in unterschiedlichen Höhen oberhalb des Unterteils 2 positioniert werden. Eine solche Höheneinstellung der Gleitfläche 5 ist bei der Kniegelenkersatzoperation zur sog. Probereposition erforderlich. Die Probereposition ist ein dem eigentlichen Kniegelenkersatz vorgelagerter Operationsschritt, in welchem die für einen funktionsgerechten Ersatz des Kniegelenks erforderlichen Abmessungen und Formen der tibialen und femoralen Implantatkomponenten bestimmt werden. Dieser anwendungsseitige Hintergrund des tibialen Probeimplantats 1 ist dem Fachmann bekannt. Es bedarf daher keiner weiteren diesbezüglichen Erklärungen.

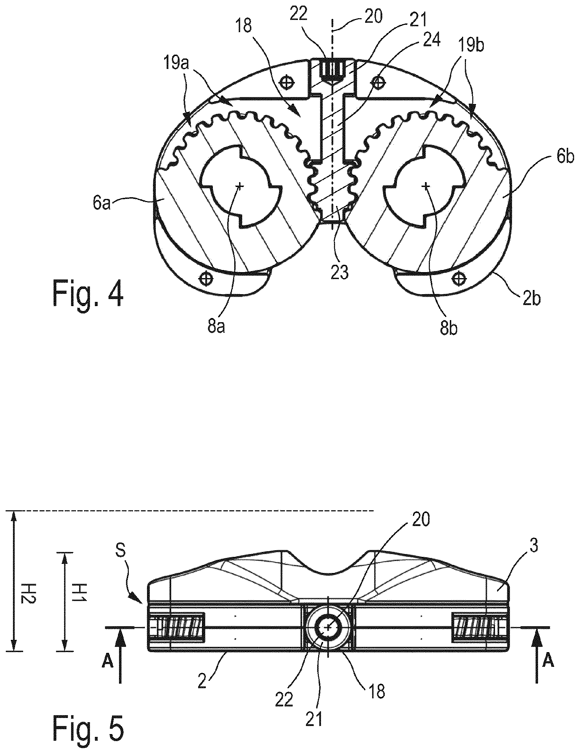

Der Höheneinstellmechanismus E ist auf noch näher beschriebene Weise mit dem Oberteil 3 und dem Unterteil 2 wirkverbunden und gestattet eine Relativverlagerung des Oberteils 3 gegenüber dem Unterteil 2 entlang der Hochrichtung Z. Das Oberteil 3 ist hierdurch zwischen unterschiedlichen Einstellpositionen in Hochrichtung H verlagerbar, wobei anhand der Fig. 1 und 5 exemplarisch eine erste Einstellposition gezeigt ist, in welcher die Gleitfläche 5 in einer ersten Höhe H1 (Fig. 5) oberhalb des Unterteils 2 positioniert ist. Die erste Höhe H1 ist anhand Fig. 5 exemplarisch zwischen einer Oberkante der Gleitfläche 5 und der Unterseite 4 des Unterteils 2 eingezeichnet. In einer zeichnerisch nicht dargestellten zweiten Einstellposition ist das Oberteil 3 in Hochrichtung Z nach oben verlagert, wobei die Gleitfläche 5 in einer zweiten Höhe H2 positioniert ist. Die zweite Höhe H2 ist in Fig. 5 schematisch angedeutet.

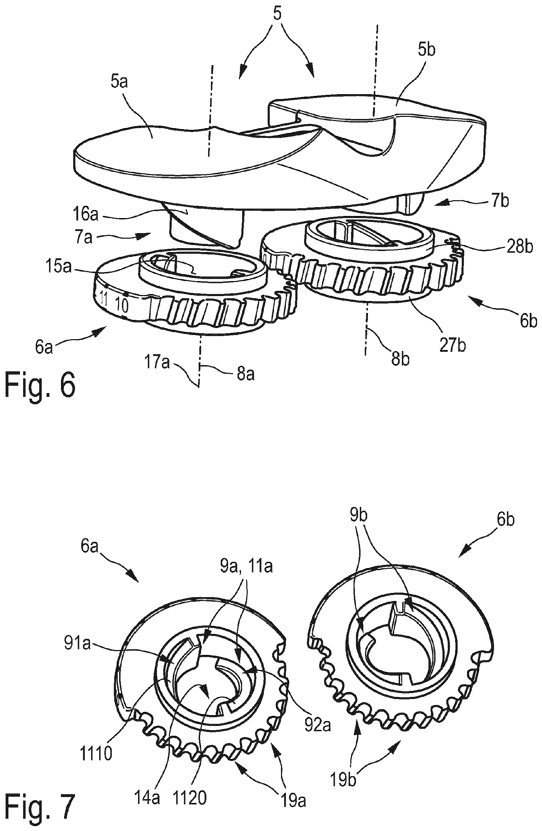

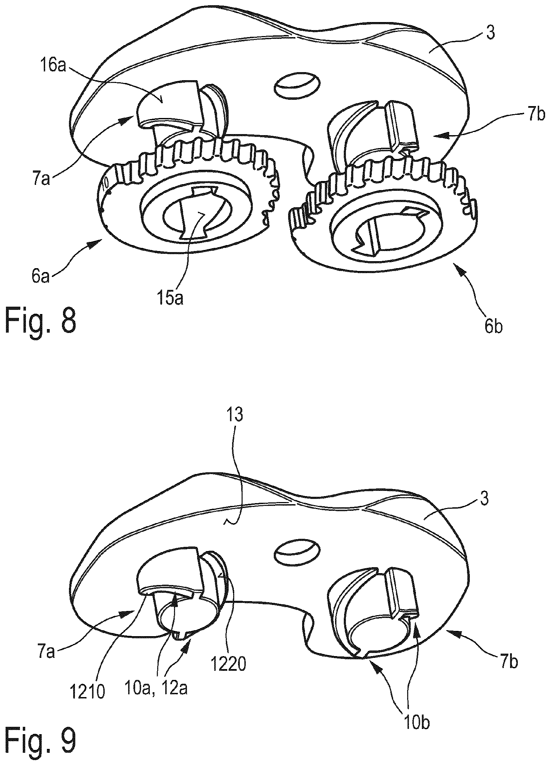

Der Höheneinstellmechanismus E weist ein Antriebsrad 6a und ein Abtriebselement 7a auf (vgl. Fig. 6 bis 9). Das Antriebsrad 6a ist um eine parallel zur Hochrichtung Z orientierte erste Drehachse 8a drehbar an dem Unterteil 2 gelagert. Weiter weist das Antriebsrad 6a eine in Hochrichtung Z ansteigende Steuerkurve 9a auf. Das Abtriebselement 7a ist fest mit dem Oberteil 3 verbunden und weist einen an der Steuerkurve 9a gleitend abgestützten Stützabschnitt 10a auf. Das Antriebsrad 6a, dessen Steuerkurve 9a, das Abtriebselement 7a und dessen Stützabschnitt 10a bilden ein Kurvengetriebe. Das Kurvengetriebe gestattet eine Übertragung der Drehbewegung des Antriebsrads 6a um die erste Drehachse 8a in eine translatorische Bewegung des Oberteils 3 entlang der Hochrichtung Z. Mit anderen Worten ausgedrückt, wird der gleitend auf der Steuerkurve 9a abgestützte Stützabschnitt 10a bei einer Drehung des Antriebsrads 6a in Hochrichtung Z nach oben gedrückt. Hierdurch kann das Oberteil 3 zum vorbeschriebenen Zweck zwischen der ersten Einstellposition und der zweiten Einstellposition verlagert und damit die Gleitfläche 5 zwischen der ersten Höhe H1 und der zweiten Höhe H2 (Fig. 5) positioniert werden. Das Antriebsrad 6a und das Abtriebselement 7a können gemäß einer jedenfalls im Bereich der Getriebetechnik üblichen Bezeichnung auch mit den Begriffen "Kurventräger" bzw. "Abgriffsglied" belegt werden.

Bei der gezeigten Ausführungsform sind dem Antriebsrad 6a auf noch näher beschriebene Weise weitere Bauteile und/oder Abschnitte des Höheneinstellmechanismus E vorgelagert, die einer manuellen und/oder werkzeuggetriebenen Betätigung dienen. Eine solche Gestaltung ist jedoch nicht zwingend. Bei einer nicht gezeigten Ausführungsform kann das Antriebsrad selbst zur manuellen Betätigung eingerichtet sein und hierfür, beispielsweise an einem Außenumfang, einen entsprechenden Betätigungsabschnitt aufweisen.

Weiter ist bei der gezeigten Ausführungsform eine in Bezug auf eine Mittellängsebene X-Z wenigstens weitgehend, vorzugsweise vollständig, spiegelsymmetrische Gestaltung des tibialen Probeimplantats 1 vorgesehen. Dementsprechend ist auch der Höheneinstellmechanismus E in Bezug auf die vertikale Mittellängsebene X-Z entsprechend spiegelsymmetrisch gestaltet. Wie anhand der Figuren gezeigt ist, weist der Höheneinstellmechanismus E zwei Antriebsräder 6a, 6b und zwei Abtriebselemente 7a, 7b auf. Das weitere Antriebsrad 6a und das weitere Abtriebselement 7a sind in Querrichtung Y von dem Antriebsrad 6a und dem Abtriebselement 7a beabstandet angeordnet. Das weitere Antriebsrad 6b ist um eine weitere erste Drehachse 8b drehbar an dem Unterteil 2 gelagert. Die weitere erste Drehachse 8b ist parallel zu der ersten Drehachse 8a orientiert. Die Form- und/oder Gestaltgebung des weiteren Antriebsrads 6b und des weiteren Abtriebselements 7a ist in Bezug auf die Mittellängsebene X-Z spiegelsymmetrisch zu dem Antriebsrad 6a und dem Abtriebselement 7a.

Es wird ausdrücklich darauf hingewiesen, dass die spiegelsymmetrische Gestaltung der vorliegenden Ausführungsform vorteilhaft, aber im Hinblick auf die Erfindung als nicht zwingend zu erachten ist. Dementsprechend ist bei einer nicht gezeigten Ausführungsform lediglich ein Antriebsrad vorgesehen, das mit einem Abtriebselement zusammenwirkt.

Zur Vermeidung von Wiederholungen wird in der nachfolgenden Beschreibung vornehmlich auf das Antriebsrad 6a und das Abtriebselement 7a im Detail Bezug genommen. Die diesbezüglich offenbarten funktionellen und gegenständlichen Merkmale gelten sinngemäß im Hinblick auf das weitere Antriebsrad 6b und das weitere Abtriebselement 7b. Umgekehrt gilt dies ebenso.

Die Steuerkurve 9a ist vorliegend durch eine mit einer konstanten Steigung wendelförmig koaxial um die erste Drehachse 8a erstreckte Steuerfläche 11a gebildet, die auch als Schrägfläche bezeichnet werden kann.

Die Steuerkurve 9a und damit auch die Steuerfläche 11a weist vorliegend disjunkte Teilabschnitte auf, nämlich einen ersten Steuerkurvenabschnitt 91a und einen zweiten Steuerkurvenabschnitt 92a bzw. einen ersten Steuerflächenabschnitt 1110 und einen zweiten Steuerflächenabschnitt 1120. Eine solche Gestaltung ist jedoch nicht zwingend. Bei einer nicht gezeigten Ausführungsform kann die Steuerkurve durch eine entlang ihrer Längserstreckung einstückig zusammenhängende Steuerfläche gebildet sein.

Grundsätzlich kann der Stützabschnitt 10a punktförmig, linienförmig oder flächig auf der Stützfläche 11a abgestützt sein. Vorliegend ist eine flächige Abstützung vorgesehen, so dass der Stützabschnitt 11a demgemäß durch eine Stützfläche 12a gebildet ist. Die Stützfläche 12a ist komplementär zu der Steuerfläche 11a gestaltet. Demnach ist die Stützfläche 12a mit einer konstanten Steigung wendelförmig koaxial um die erste Drehachse 8a erstreckt. Zudem ist eine disjunkte Gestaltung mit zwei Stützflächenabschnitten 1210, 1220 vorgesehen.

Das Abtriebselement 7a ragt vorliegend entlang der Hochrichtung Z von oben nach unten von einer Unterseite 13 des Oberteils 3 ab. Dabei weist das Abtriebselement 7a eine zapfenförmige Grundform auf. Das Abtriebselement 7a ist einstückig an die Unterseite 13 des Oberteils 3 angeformt. Bei einer weiteren Ausführungsform kann das Antriebselement separat von dem Oberteil im Übrigen gefertigt und auf grundsätzlich bekannte Weise an das Oberteil angefügt sein.

Um einen möglichst einfachen Austausch des Oberteils 3 gegen ein unterschiedlich gestaltetes Oberteil, das beispielsweise eine unterschiedlich gestaltete Gleitfläche aufweist, zu ermöglichen, ist vorliegend eine Steckverbindung zwischen dem Oberteil 3 und dem Unterteil 2 vorgesehen. Diese Steckverbindung ist auf einfache Weise manuell lösbar, indem das Oberteil 3 in Hochrichtung Z nach oben von dem Unterteil 2 abgezogen wird. Die Steckverbindung ist dabei zwischen dem Antriebsrad 6a und dem Abtriebselement 7a ausgebildet. Hierfür weist das Antriebsrad 6a eine koaxial zu der ersten Drehachse 8a erstreckte Zylinderbohrung 14a auf. Die Zylinderbohrung 14a ist vorliegend als Durchgangsbohrung gestaltet. Die Zylinderbohrung 14a weist eine Zylindermantelfläche 15a auf. Das Abtriebselement 7a weist einen zu der Zylinderbohrung 14a komplementären Steckzylinder auf bzw. fungiert als ein solcher Steckzylinder und ist dementsprechend mit einer komplementären Zylindermantelfläche 16a versehen. Die Zylindermantelfläche 16 des Abtriebselements 7a wirkt bei einer Drehbetätigung des Antriebsrads 6a in Umfangsrichtung um die erste Drehachse 8a und axial entlang der Hochrichtung Z gleitbeweglich mit der Zylindermantelfläche 15a der Zylinderbohrung 14a zusammen.

Bei der gezeigten Ausführungsform ist die Steuerkurve 9a als eine Art radialer Vorsprung in der Zylinderbohrung 14a angeordnet. Bei einer nicht gezeigten Ausführungsform ist die Steuerkurve stattdessen in Axialrichtung versetzt zu der Durchgangsbohrung 14a an einer Oberseite des Antriebsrads 9a angeordnet. Der Stützabschnitt 10a ist durch einen radialen Rücksprung der Zylindermantelfläche 16a des Steckzylinders gebildet. Bei einer nicht gezeigten Ausführungsform kann der Stützabschnitt örtlich getrennt von dem Steckzylinder ausgebildet sein.

Der durch das Abtriebselement 7a gebildete Steckzylinder ist in Hochrichtung von oben nach unten entlang der ersten Drehachse 8a in die Zylinderbohrung 14a eingesteckt. Die erste Drehachse 8a fällt bei der gezeigten Ausführungsform damit mit einer Steckachse 17a der Steckverbindung zusammen.

Aufgrund der spiegelsymmetrischen Gestaltung der vorliegenden Ausführungsform ist die Steckverbindung zwischen dem Oberteil 3 und dem Unterteil 2 vorliegend zudem zwischen dem weiteren Antriebsrad 6b und dem weiteren Abtriebselement 7b ausgebildet. Diesbezüglich gilt das zu der Steckverbindung zwischen dem Antriebsrad 6a und dem Abtriebselement 7a Gesagte sinngemäß. Auf eine gesonderte Bezugnahme auf die zur Bewegungsübertragung und Ausbildung der Steckverbindung erforderlichen Abschnitte an dem weiteren Antriebsrad 6b und dem weiteren Abtriebselement 7b wird der gebotenen Kürze wegen verzichtet. Stattdessen wird auf die diesbezüglichen Erläuterungen im Zusammenhang mit dem Antriebsrad 6a und dem Abtriebselement 7a verwiesen.

Der Höheneinstellmechanismus E weist vorliegend zudem ein dem Kurvengetriebe vorgeschaltetes Schneckengetriebe 18, 19a, 19b auf. Das Schneckengetriebe 18, 19a, 19b umfasst eine Schneckenantriebswelle 18 und einen zu der Schneckenantriebswelle 18 komplementär verzahnten Außenumfangsabschnitt 19a des Antriebsrads 6a.

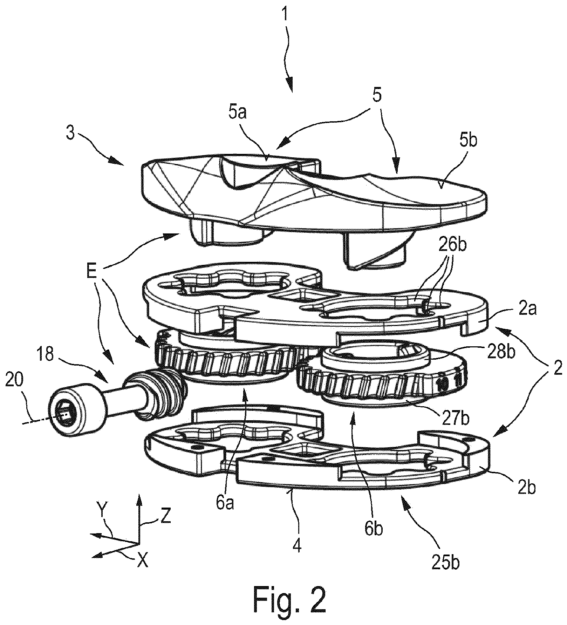

Die Schneckenantriebswelle 18a ist um eine senkrecht zur Hochrichtung Z orientierte zweite Drehachse 20 (Fig. 2) drehbar an dem Unterteil 2 gelagert und wirkt zur Bewegungs- und Kraftübertragung mit dem verzahnten Außenumfangsabschnitt 19a zusammen (Fig. 4). Bei der gezeigten Ausführungsform wirkt die Schneckenantriebswelle 18 zudem mit einem komplementär verzahnten Außenumfangsabschnitt 19b des weiteren Antriebsrads 6b zusammen. Die Schneckenantriebswelle 18 ragt in Längsrichtung X zwischen die beiden Antriebsräder 6a, 6b. Bei einer Drehbewegung der Schneckenantriebswelle 18 um die dritte Drehachse 20 werden die beiden Antriebsräder 6a, 6b zueinander gegenläufig um die jeweilige erste Drehachse 8a, 8b angetrieben.

Das auf diese Weise gebildete Schneckengetriebe 18, 19a, 19b ist vorliegend selbsthemmend gestaltet. Demnach ist das Schneckengetriebe 18, 19a, 19b ausschließlich mittels einer antriebsseitigen Drehbewegung der Schneckenantriebswelle 18 antreibbar. Ein Antrieb mittels einer von den Antriebsrädern 6a, 6b ausgehenden Bewegung ist demgegenüber reibungs- und/oder übersetzungsbedingt gehemmt und somit nicht möglich.

Die Schneckenantriebswelle 18 weist einen Drehbetätigungsabschnitt 21 auf, der bei der gezeigten Ausführungsform mit einer Werkzeugfläche 22 versehen ist. Die Werkzeugfläche 22 ist eine Innensechskantfläche, die zum Zusammenwirken mit einem üblichen für den medizinischen Gebrauch vorgesehenen Innensechskantschlüssel eingerichtet ist. Bei einer nicht gezeigten Ausführungsform ist anstelle der Werkzeugfläche 22 eine durch den Drehbetätigungsabschnitt gebildete Kurbel, ein Betätigungsrad oder dergleichen vorgesehen.

Der Drehbetätigungsabschnitt 21 ist einends der Schneckenantriebswelle 18 angeordnet und im verwendungsbereit zusammengefügten Zustand des tibialen Probeimplantats 1 von außen zugänglich. Andernends weist die Schneckenantriebswelle 18 einen Verzahnungsabschnitt 23 auf, der bei einer Drehbewegung der Schneckenantriebswelle 18 mit den verzahnten Außenumfangsabschnitten 19a, 19b zusammenwirkt. Zwischen dem Verzahnungsabschnitt 23 und dem Drehbetätigungsabschnitt 21 ist ein Schaftabschnitt 24 in axialer Richtung erstreckt.

Das Antriebsrad 6a, das weitere Antriebsrad 6b und die Schneckenantriebswelle 18 sind zwischen der Oberschale 2a und der Unterschale 2b des Unterteils 2 gehalten, wobei insbesondere zur Lagerung der Antriebsräder 6a, 6b entsprechende Gleitlagerflächen vorgesehen sind, die in den Figuren zur vereinfachten zeichnerischen Darstellung lediglich in Bezug auf das weitere Antriebsrad 6b bezeichnet sind. Im Hinblick auf die Gleitlagerung des Antriebsrads 6a gilt sinngemäß das zu dem weiteren Antriebsrad 6b Gesagte. Die dem weiteren Antriebsrad 6b zugeordnete Gleitlagerfläche ist durch eine koaxial zu der weiteren Drehachse 8b durch das Unterteil 2 erstreckte Durchgangsbohrung 25b gebildet. Die Durchgangsbohrung 25b weist - ausgehend von einer gedachten kreiszylindrischen Formgebung - mehrere radiale Ausbuchtungen 26b auf. Die Ausbuchtungen 26b können auch als Spülausbuchtungen bezeichnet werden und ermöglichen eine vereinfachte und somit besonders hygienische Durchspülbarkeit des Unterteils 2 mit einer Flüssigkeit. Das weitere Antriebsrad 6b weist einen unteren Radialbund 27b und einen oberen Radialbund 28b auf, die zur gleitenden Anlage an den entsprechenden Gleitlagerflächen der Oberschale 2a bzw. der Unterschale 2b vorgesehen sind.

Weiter ist eine zwischen dem Antriebsrad 6a und dem Unterteil 2 ausgebildete Skalenanzeige S vorgesehen, deren Anordnung anhand Fig. 5 ersichtlich ist. Die Skalenanzeige S weist ein als Ablesestrich gestaltetes Ableseelement auf, das sowohl an der Oberschale 2a als auch an der Unterschale 2b angeordnet ist. Zudem ist eine in Fig. 6 ersichtliche Skale mit einer Ziffernfolge vorgesehen. Die Skalenanzeige S ermöglicht ein Ablesen der Einstellposition des Oberteils.

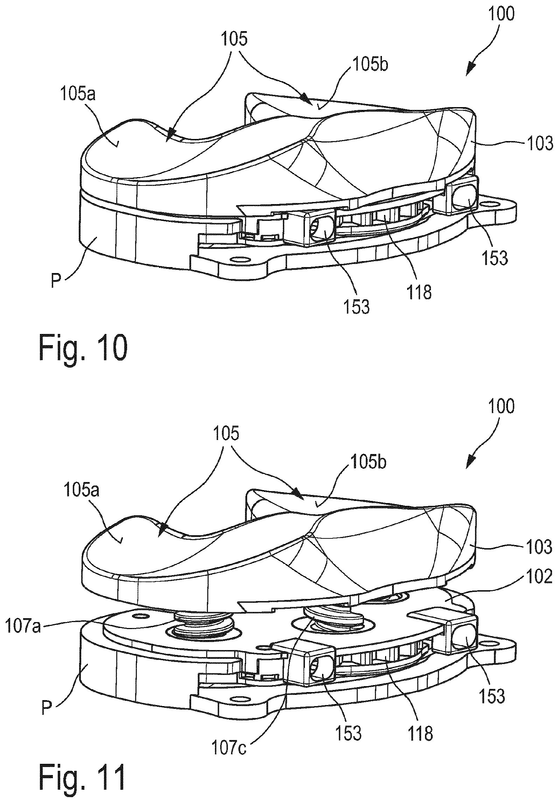

Anhand der Fig. 10 bis 18 ist eine weitere Ausführungsform eines erfindungsgemäßen tibialen Probeimplantats 100 gezeigt. Die grundsätzliche Gestaltung und Funktionsweise des tibialen Probeimplantats 100 sind in weiten Teilen übereinstimmend mit der Gestaltung und Funktionsweise des tibialen Probeimplantats 1 nach den Fig. 1 bis 9. Nachfolgend wird daher in erster Linie auf wesentliche Unterschiede des tibialen Probeimplantats 100 eingegangen. Im Übrigen wird zur Vermeidung von Wiederholungen auf die Beschreibung des tibialen Probeimplantats 1 verwiesen.

Das tibiale Probeimplantat 100 weist wiederum ein Unterteil 102, ein Oberteil 103 und einen Höheneinstellmechanismus E` auf.

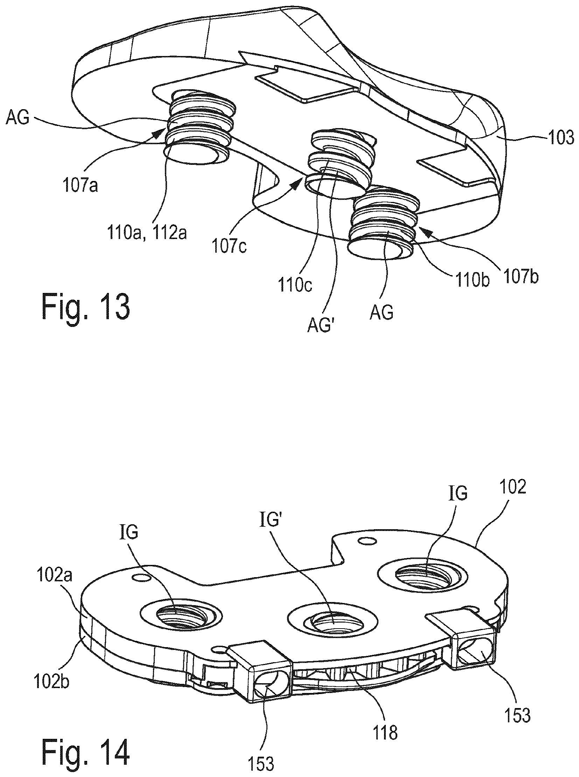

Das Unterteil 102 (Fig. 14) ist vorliegend zur mittelbaren tibialen Fixierung eingerichtet und wirkt dementsprechend mit einem Tibia-Probeplateau P zusammen. In einem verwendungsbereiten Zustand ist das Unterteil 102 mit seiner Unterseite 104 voran in eine hierfür eingerichtete und nicht näher bezeichnete Aufnahmeaussparung des Tibia-Probeplateaus P eingesetzt.

In Übereinstimmung mit dem Unterteil 2 weist das Unterteil 102 eine plattenförmige Grundform und eine zweischalige Gestaltung mit einer Oberschale 102a und einer Unterschale 102b auf.

In Übereinstimmung mit dem Oberteil 3 weist das Oberteil 103 eine oberseitig angeordnete Gleitfläche 105 mit zwei in Querrichtung Y voneinander beabstandeten Teilgleitflächen 105a, 105b auf.

Der Höheneinstellmechanismus E' ist - in grundsätzlicher Übereinstimmung mit dem Höheneinstellmechanismus E - einerseits mit dem Oberteil 103 und andererseits mit dem Unterteil 102 wirkverbunden und gestattet eine Relativverlagerung des Oberteils 103 gegenüber dem Unterteil 102 entlang der Hochrichtung Z. Dabei zeigt Fig. 10 exemplarisch eine erste Einstellposition, die auch als untere Endlage bezeichnet werden kann. In der unteren Endlage weist das tibiale Probeimplantat 100 seine minimale Gesamtbauhöhe und/oder Dicke auf. Fig. 11 zeigt eine zweite Einstellposition, die auch als obere Endlage bezeichnet werden kann. In dieser nimmt das tibiale Probeimplantat 100 seine maximale Gesamtbauhöhe und/oder Dicke ein.

Der Höheneinstellmechanismus E' weist wiederum wenigstens ein Antriebsrad 106a und ein Abtriebselement 107a auf. Das Antriebsrad 106a ist um eine parallel zur Hochrichtung Z orientierte nicht näher bezeichnete Drehachse drehbar an dem Unterteil 102 gelagert und weist eine in Hochrichtung Z ansteigende Steuerkurve 109a auf. Die Steuerkurve 109a ist bei der gezeigten Ausführungsform durch eine mit einer konstanten Steigung wendelförmig koaxial um die besagte Drehachse erstreckte Steuerfläche 111a gebildet.

Vorliegend sind die Steuerkurve 109a und/oder die Steuerfläche 111a in Gestalt eines Innengewindes IG ausgebildet. Das Innengewinde IG ist in eine Zylinderbohrung 114a des Antriebsrads 106a eingebracht. Die Zylinderbohrung 114a ist koaxial zu der Drehachse des Antriebsrads 106a erstreckt.

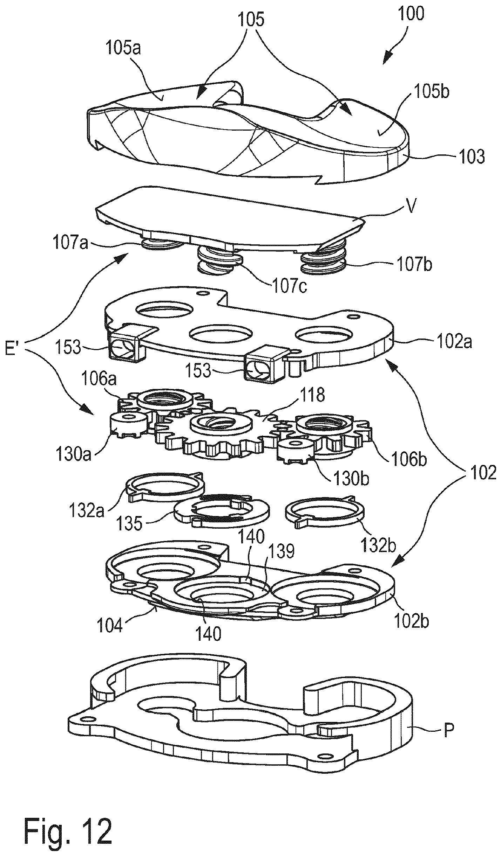

Das wenigstens eine Abtriebselement 107a ist fest mit dem Oberteil 103 verbunden. Im Unterschied zu dem Abtriebselement 7a des tibialen Probeimplantats 1 ist das Abtriebselement 107a nicht etwa einstückig an dem Oberteil 103 ausgebildet. Stattdessen ist ein dem Oberteil 103 zugeordnetes Verbindungselement V vorhanden (Fig. 12), an welchem das Abtriebselement 107a ausgebildet ist. Das Verbindungselement V ist auf eine dem Fachmann grundsätzlich bekannte Weise in Längsrichtung lösbar und in Quer- sowie Hochrichtung Y, Z formschlüssig mit dem Oberteil 103 zusammengesteckt. Das wenigstens eine Abtriebselement 107a weist - in grundsätzlicher Übereinstimmung mit dem Abtriebselement 7a - einen Stützabschnitt 110a auf, der gleitend an der Steuerkurve 109a abgestützt ist. Bei der gezeigten Ausführungsform ist der Stützabschnitt 110a durch eine mit einer konstanten Steigung wendelförmig erstreckte Stützfläche 112a gebildet.

Der Stützabschnitt 110a und/oder die Stützfläche 112a sind als Außengewinde AG gestaltet. Das Außengewinde AG ist komplementär zu dem Innengewinde IG. Vereinfacht ausgedrückt bilden das Innengewinde IG und das Außengewinde AG eine Art Gewindespindel, mittels derer die Drehbewegung des Antriebsrads 106a in eine translatorische Bewegung des Abtriebselements 107a und damit auch des Oberteils 103 umsetzbar ist.

Vereinfacht ausgedrückt kann das Abtriebselement 107a als Schraube und das Antriebsrad 106a als Mutter bezeichnet werden.

Der Höheneinstellmechanismus E' ist - ähnlich wie der Höheneinstellmechanismus E - wenigstens weitgehend, vorzugsweise vollständig, spiegelsymmetrisch gestaltet. Der Höheneinstellmechanismus E' weist wiederum wenigstens zwei Antriebsräder 106a, 106b und wenigstens zwei Abtriebselemente 107a, 107b auf. Diese werden nachfolgend auch als erstes Antriebsrad 106a, zweites Antriebsrad 106b, erstes Abtriebselement 107a und zweites Abtriebselement 107b bezeichnet. Hinsichtlich der Gestaltung und Funktionsweise des zweiten Antriebsrads 106b gilt sinngemäß das bereits zu dem ersten Antriebsrad 106a Gesagte. Entsprechendes gilt sinngemäß im Hinblick auf die Funktionsweise und Gestaltung des zweiten Abtriebselements 107b. Zur Vermeidung von Wiederholungen wird dementsprechend auf die Offenbarung zu dem ersten Antriebsrad 106a und dem ersten Abtriebselement 107a verwiesen.

Den Antriebsrädern 106a, 106b ist ein Steuerrad 118 vorgeschaltet. Das Steuerrad 118 ist um eine parallel zu den Drehachsen der Antriebsräder 106a, 106b orientierte Drehachse drehbar an dem Unterteil 102 drehbar gelagert. Die beiden Antriebsräder 106a, 106b und das Steuerrad 118 weisen jeweils eine Stirnverzahnung 119a, 119b, 119 auf. Die Stirnverzahnung 119 des Steuerrads 118 ist mit der Stirnverzahnung 119a des ersten Antriebsrads 106a und mit der Stirnverzahnung 119b des zweiten Antriebsrads 106b verzahnt. Hierdurch sind die jeweiligen Drehbewegungen miteinander zwangsgekoppelt und/oder synchronisiert. Eine Drehbewegung des Steuerrads 118 entgegen dem Uhrzeigersinn bewirkt eine jeweils im Uhrzeigersinn gerichtete Drehbewegung des ersten Antriebsrads 106a und des zweiten Antriebsrads 106b.

Die Stirnverzahnungen 119a, 119b der beiden Antriebsräder 106a, 106b sind bei der gezeigten Ausführungsform identisch im Hinblick auf ihre jeweilige Zähnezahl und die sonstigen Verzahnungsmerkmale. Die Stirnverzahnung 119 des Steuerrads 118 weist eine größere Zähnezahl als die Stirnverzahnungen 119a, 119b auf. Ein nicht näher bezeichneter Außendurchmesser des Steuerrads 118 ist größer als der Außendurchmesser der Antriebsräder 106a, 106b.

Das Steuerrad 118 ist bei der gezeigten Ausführungsform zur mittelbaren manuellen Betätigung eingerichtet, was nachfolgend noch näher beschrieben wird. Bei einer weiteren Ausführungsform ist das Steuerrad zur unmittelbaren manuellen Drehbetätigung eingerichtet.

Das Steuerrad 118 weist vorliegend in Übereinstimmung mit den Antriebsrädern 106a, 106b ein Innengewinde IG' auf. Das Innengewinde IG' bildet in Entsprechung zu den Innengewinden IG eine nicht näher bezeichnete Steuerkurve, genauer: eine mit konstanter Steigung wendelförmig erstreckte Steuerfläche. Das Innengewinde IG' des Steuerrads 118 wirkt mit einem Außengewinde AG` eines weiteren Abtriebselements 107c zusammen. Das weitere Abtriebselement 107c wird nachfolgend auch als drittes Abtriebselement 110c bezeichnet. Das Außengewinde AG` des dritten Abtriebselements 107c bildet wiederum einen Stützabschnitt, genauer: eine mit einer konstanten Steigung wendelförmig erstreckte Stützfläche.

Das Steuerrad 118 fungiert insoweit gleichsam als zusätzliches, drittes Antriebsrad. Das Innengewinde IG' und das Außengewinde AG` sind zueinander komplementär. Der Drehsinn des Innengewindes IG' ist entgegengesetzt zu dem Drehsinn der Innengewinde IG der Antriebsräder 106a, 106b. Entsprechendes gilt sinngemäß für den Drehsinn des Außengewindes AG`. Zudem weist das Innengewinde IG' eine Gewindesteigung auf, die unterschiedlich zu der Gewindesteigung der Innengewinde IG der beiden Antriebsräder 106a, 106b ist. Dementsprechend ist auch die Gewindesteigung des Außengewindes AG` unterschiedlich zu der Gewindesteigung der Außengewinde AG der Abtriebselemente 107a, 107b.

Das Steuerrad 118 ist in Querrichtung Y mittig zwischen dem ersten Antriebsrad 106a und dem zweiten Antriebsrad 106b angeordnet.

Das tibiale Probeimplantat 100 weist ein erstes Anzeigerad 130a und ein zweites Anzeigerad 130b auf. Das erste Anzeigerad 130a ist dem ersten Antriebsrad 106a zugeordnet und bewegungsübertragend mit diesem gekoppelt. Das zweite Anzeigerad 130b ist dem zweiten Antriebsrad 106b zugeordnet und bewegungsübertragend mit diesem gekoppelt. Bei einer weiteren Ausführungsform ist lediglich ein einziges Anzeigerad vorhanden. Die Gestaltung und Funktion der Anzeigeräder 130a, 130b ist identisch, so dass zur Vermeidung von Wiederholungen nachfolgend lediglich auf das erste Anzeigerad 130a eingegangen wird. Das diesbezüglich Offenbarte gilt sinngemäß auch für das zweite Anzeigerad 130b.

Das erste Anzeigerad 130a ist drehbeweglich an dem Unterteil 102 gelagert und weist eine zeichnerisch nicht im Detail dargestellte Skale S` auf. Die Skale S' ist auf einen Außenumfang 131a des ersten Anzeigerads 130a angeordnet. Die Skale S' kann beispielsweise durch eine Abfolge von Teilungsstrichen oder Ziffern gebildet sein. Die Skale S` gestattet ein Ablesen der Einstellposition des Oberteils 103. Dies in Entsprechung zu der Skale S des tibialen Probeimplantats 1 (Fig. 5).

Durch die bewegungsübertragende Kopplung wird eine Drehbewegung des ersten Antriebsrads 106a in eine Drehbewegung des ersten Anzeigerads 130a umgesetzt. Die Bewegungsübertragung kann kontinuierlich oder intermittierend, das heißt: schrittweise, ausgebildet sein. Bei der gezeigten Ausführungsform ist Letzteres der Fall.

Durch die noch näher erläuterte intermittierende Bewegungsübertragung bewirkt eine kontinuierliche Drehbewegung des ersten Antriebsrads 106a eine schrittweise Drehbewegung des Anzeigerads 130a. Die intermittierende Bewegungsübertragung erfolgt vorliegend mittels eines ersten Mitnehmerelements 132a. Dem zweiten Antriebsrad 106b und dem zweiten Anzeigerad 130b ist ein zweites Mitnehmerelement 132b zugeordnet.

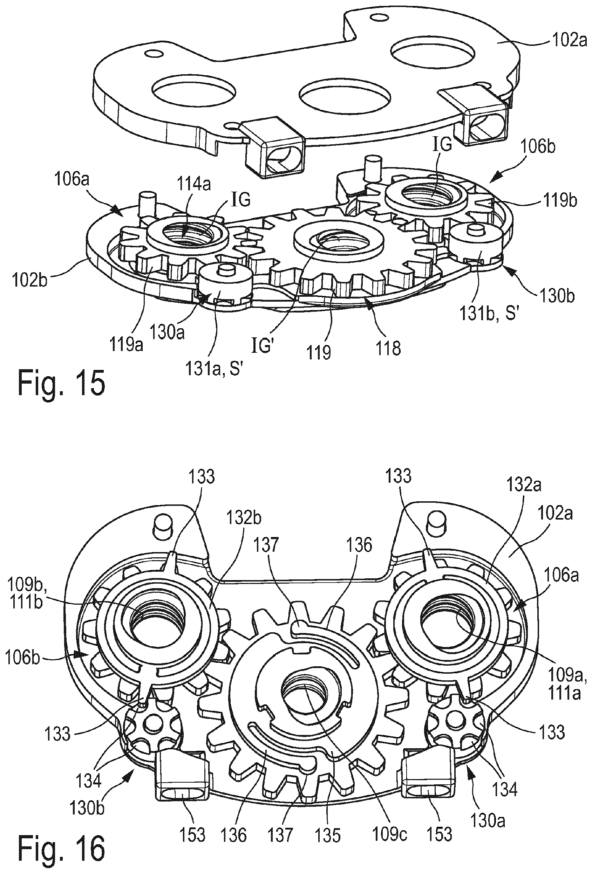

Das erste Mitnehmerelement 132a ist drehfest mit dem ersten Antriebsrad 106a verbunden. Vorliegend ist das erste Mitnehmerelement 132a ringförmig gestaltet, koaxial zu dem ersten Antriebsrad 106a ausgerichtet und auf nicht näher gezeigte Weise form-, kraft- und/oder stoffschlüssig an einer Unterseite des ersten Antriebsrads 106a festgelegt. Das erste Mitnehmerelement 132a wirkt zur Bewegungsübertragung mit einem hierfür vorgesehenen Abschnitt des Anzeigerads 130a zusammen. Vorliegend weist das erste Mitnehmerelement 132a wenigstens einen, genauer: zwei, in radialer Richtung nach außen abragende Vorsprünge 133 auf. Diese sind vorliegend um 180° in Umfangsrichtung des ersten Mitnehmerelements 132a zueinander versetzt angeordnet. Das erste Anzeigerad 130a weist an seiner nicht näher bezeichneten Unterseite in radialer Richtung zurückspringende Rücksprünge 134 auf (Fig. 16). Zur intermittierenden Bewegungsübertragung greifen die Vorsprünge 133 in die Rücksprünge 134 ein.

Bei der gezeigten Ausführungsform bewirkt eine (kontinuierliche) vollständige Umdrehung des ersten Antriebsrads 106a um 360° eine schrittweise Drehbewegung des ersten Anzeigerads 130a um zwei Inkremente. Da vorliegend genau sechs um jeweils 60° zueinander versetzt angeordnete Rücksprünge 134 vorhanden sind, wird das erste Anzeigerad 130a dementsprechend um jeweils zwei Schritte à 60° drehbewegt.

Es versteht sich, dass hinsichtlich der Gestaltung und Funktion des zweiten Mitnehmerelements 132b und des zweiten Anzeigerads 130b sinngemäß das vorstehend Gesagte gilt.

Dem Steuerrad 118 ist ein Rastelement 135 zugeordnet. Das Rastelement bildet auf noch näher beschriebene Weise eine lösbare Rastverbindung mit dem Unterteil 102, genauer: dessen Unterschale 102b, aus. Die Rastverbindung ist überrastbar und wird in definierten Drehstellungen des Steuerrads 118 und damit der beiden Antriebsräder 106a, 106b etabliert. Hierdurch erhält ein Benutzer bei der Einstellung des Höheneinstellmechanismus E' eine taktile und/oder akustische Rückmeldung, sobald eine definierte Drehstellung und damit Höheneinstellung des Oberteils 103 erreicht ist.

Bei der gezeigten Ausführungsform ist das Rastelement 135 ringförmig gestaltet und auf nicht näher gezeigte Weise kraft-, form- und/oder stoffschlüssig an eine Unterseite des Steuerrads 118 angefügt. Dabei ist das Rastelement 135 koaxial zu dem Steuerrad 118 ausgerichtet und gemeinsam mit diesem um dessen Drehachse drehbar. Das Rastelement 135 wirkt mit einem an dem Unterteil angeordneten komplementären Gegenrastelement 139 zusammen (Fig. 12), das vorliegend als kreiszylindrische Aussparung ausgebildet ist. Die kreiszylindrische Aussparung ist in eine Oberseite der Unterschale 102b eingesenkt. In betriebsfertig montiertem Zustand ist das Mitnehmerelement 135 in der kreiszylindrischen Aussparung 139 aufgenommen. Das Mitnehmerelement 135 weist bei der gezeigten Ausführungsform zwei um 180° versetzt angeordnete Federarme 136 auf, an deren Stirnenden jeweils ein Rastvorsprung 137 ausgebildet ist. Die Rastvorsprünge 137 sind in radialer Richtung federbeweglich und wirken zum Verrasten mit Rastaussparungen 140 zusammen.

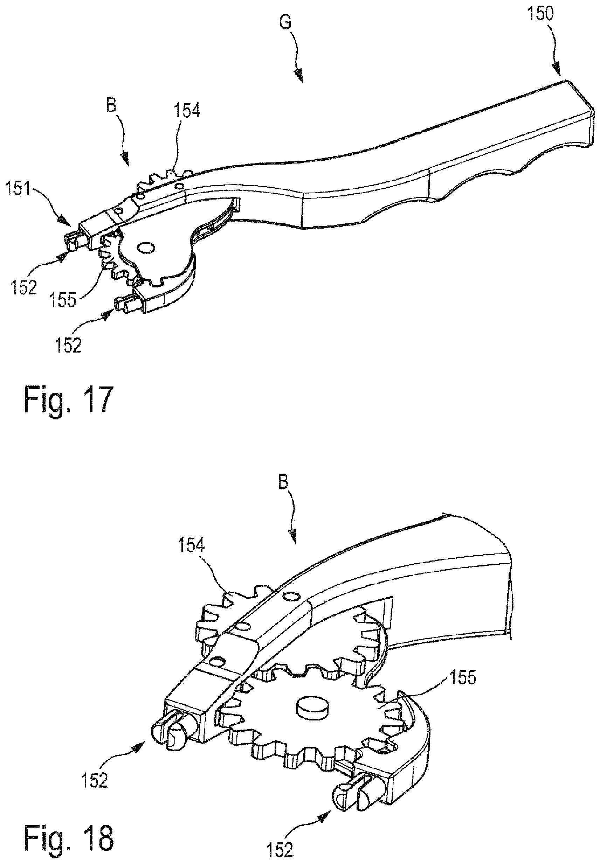

Das tibiale Probeimplantat 100 weist zudem einen anhand der Fig. 17 und 18 gezeigten Handgriff G auf, der auf noch näher beschriebene Weise lösbar mit dem Unterteil 102 verbindbar ist. Der Handgriff G dient zum einen einer vereinfachten Handhabung des Unterteils 102. Zum anderen weist der Handgriff G eine Betätigungsmechanik B auf, die einer vereinfachten und besonders ergonomischen Betätigung des Höheneinstellmechanismus E' dient.

Der Handgriff G ist zwischen einem proximalen Ende 150 und einem distalen Ende 151 längserstreckt. Die Betätigungsmechanik B ist vorliegend im Bereich des distalen Endes 151 angeordnet. Zur lösbaren Verbindung mit dem Unterteil 102 weist der Handgriff G vorliegend zwei an dem distalen Ende 151 angeordnete Verbindungselemente jeweils in Form eines Rastelements 152 auf. Bei einer nicht gezeigten Ausführungsform ist lediglich ein Rastelement vorhanden. Die beiden Rastelemente 152 sind in Querrichtung Y zueinander beabstandet angeordnet und wirken in einem mit dem Unterteil 102 verbundenen Zustand mit jeweils einem komplementären Verbindungselement in Form eines Gegenrastelements 153 zusammen. Die Gegenrastelemente 153 sind dementsprechend in Querrichtung Y zueinander beabstandet angeordnet und vorliegend zudem beidseits des Steuerrads 118 positioniert.

Bei der gezeigten Ausführungsform sind die Rastelemente 152 jeweils als männliches Rastelement und die Gegenrastelemente 153 sind jeweils als weibliches Rastelement beziehungsweise als Rastaufnahme gestaltet. Die Rastelemente 152 ragen in distaler Richtung von dem distalen Ende 151 des Handgriffs G ab. Die komplementären Rastelemente 153 sind in distaler Richtung des Handgriffs G in das Unterteil 102 hinein erstreckt. Die Gegenrastelemente 153 sind an der Oberschale 102a ausgebildet.

Die Betätigungsmechanik B weist ein Betätigungsrad 154 auf, das in einem mit dem Unterteil 102 verbundenen Zustand des Handgriffs G wenigstens mittelbar mit den Antriebsrädern 106a, 106b kraft- und bewegungsübertragend wirkverbunden ist. Das Betätigungsrad 154 ist um eine nicht näher bezeichnete Drehachse drehbar an dem Handgriff G gelagert und kann auch als Daumenrad bezeichnet werden. Eine Drehung des Betätigungsrads 154 bewirkt eine Drehung der Antriebsräder 106a, 106b und damit eine entsprechende Höheneinstellung des Oberteils 103.

Bei der gezeigten Ausführungsform ist das Betätigungsrad mittelbar über ein drehbar an dem Handgriff G gelagertes Übertragungsrad 155 und das Steuerrad 118 mit den Antriebsrädern 106a, 106b wirkverbunden. Hierzu ist das Betätigungsrad 154 mit einer nicht näher bezeichneten Stirnverzahnung versehen, die mit einer Stirnverzahnung des Übertragungsrads 155 in Eingriff steht. Die Stirnverzahnung des Übertragungsrads 155 ist ihrerseits mit der Stirnverzahnung 119 des Steuerrads 118 verzahnt. Beim Verrasten des Handgriffs G mit dem Unterteil 102 gelangt die Verzahnung des Übertragungsrads 155 mit der Verzahnung 119 des Steuerrads 118 in Eingriff. Beim Abziehen des Handgriffs G von dem Unterteil 102 wird die Verzahnung zwischen dem Übertragungsrad 155 und dem Steuerrad 118 gelöst.

## Patentansprüche

1. Tibiales Probeimplantat (1, 100) zur Verwendung bei einer Kniegelenkersatzoperation, aufweisend
- ein Unterteil (2, 102), das zur tibialen Fixierung vorgesehen ist,
- ein in Hochrichtung (Z) oberhalb des Unterteils (2, 102) angeordnetes Oberteil (3, 103) mit einer oberseitig angeordneten Gleitfläche (5, 105), die zum gleitenden Zusammenwirken mit einer femoralen Komponente vorgesehen ist,
- einen mit dem Oberteil (3, 103) und dem Unterteil (2, 102) wirkverbundenen Höheneinstellmechanismus (E, E'), mittels dessen das Oberteil (3, 103) relativ zu dem Unterteil (2, 102) in Hochrichtung (Z) geführt verlagerbar ist zwischen
- einer ersten Einstellposition, in welcher die Gleitfläche (5, 105) in einer ersten Höhe (H1) oberhalb des Unterteils (2, 102) positioniert ist, und
- einer zweiten Einstellposition, in welcher die Gleitfläche (5, 105) in einer zweiten Höhe (H2) oberhalb des Unterteils (2, 102) positioniert ist,
- wobei der Höheneinstellmechanismus (E, E') ein Kurvengetriebe aufweist mit wenigstens einem Antriebsrad (6a, 6b, 106a, 106b), das um eine parallel zur Hochrichtung (Z) orientierte erste Drehachse (8a, 8b) drehbar an dem Unterteil (2, 102) gelagert ist und eine in Hochrichtung (Z) ansteigende Steuerkurve (9a, 9b, 109a, 109b) aufweist, und mit wenigstens einem Abtriebselement (7a, 7b, 107a, 107b), das fest mit dem Oberteil (3, 103) verbunden ist und einen an der Steuerkurve (9a, 9b, 109a, 109b) gleitend abgestützten Stützabschnitt (10a, 10b, 110a, 110b) aufweist, wodurch das Oberteil (3, 103) mittels einer Drehbewegung des Antriebsrads (6a, 6b, 106a, 106b) zwischen der ersten Einstellposition und der zweiten Einstellposition verlagerbar ist,
- **dadurch gekennzeichnet, dass** zwischen dem Unterteil (2) und dem wenigstens einen Antriebsrad (6a, 6b) eine Skalenanzeige (S) ausgebildet ist, die ein Ableseelement und eine Skale aufweist und ein Ablesen der Einstellposition des Oberteils (3) und/oder der Höhe (H1, H2) der Gleitfläche (5) gestattet, oder
- dass das wenigstens eine Antriebsrad (106a, 106b) bewegungsübertragend mit wenigstens einem an dem Unterteil (102) drehbeweglich gelagerten Anzeigerad (130a, 130b) gekoppelt ist, wobei das Anzeigerad (130a, 130b) eine Skale (S') aufweist, die ein Ablesen der Einstellposition des Oberteils (103) und/oder der Höhe der Gleitfläche (105) gestattet.

2. Tibiales Probeimplantat (1, 100) nach Anspruch 1, **dadurch gekennzeichnet, dass** die Steuerkurve (9a, 109a) durch eine mit einer konstanten Steigung wendelförmig koaxial um die erste Drehachse (8a) erstreckte Steuerfläche (11a, 111a) gebildet ist.

3. Tibiales Probeimplantat (1, 100) nach Anspruch 1 oder 2, **dadurch gekennzeichnet, dass** der Stützabschnitt (10a, 110a) durch eine mit einer konstanten Steigung wendelförmig koaxial um die erste Drehachse (8a, 108a) erstreckte Stützfläche (12a, 112a) gebildet ist.

4. Tibiales Probeimplantat (1) nach einem der vorhergehenden Ansprüche, **dadurch gekennzeichnet, dass** das Oberteil (3) und das Unterteil (2) mittels einer zwischen dem wenigstens einen Antriebsrad (6a) und dem wenigstens einen Abtriebselement (7a) ausgebildeten Steckverbindung entlang einer Steckachse (17a) relativ zueinander gleitbeweglich lösbar zusammengesteckt sind, wobei die Steckachse (17a) koaxial zu der ersten Drehachse (8a) orientiert ist, insbesondere wobei das wenigstens eine Antriebsrad (6a) eine koaxial zu der ersten Drehachse (8a) erstreckte Zylinderbohrung (14a) aufweist, in welche ein komplementärer Steckzylinder (16a) des wenigstens einen Abtriebselements (7a) unter Ausbildung der Steckverbindung lösbar eingesteckt ist.

5. Tibiales Probeimplantat (1) nach einem der vorhergehenden Ansprüche, **dadurch gekennzeichnet, dass** der Höheneinstellmechanismus (E) ein dem Kurvengetriebe vorgeschaltetes Schneckengetriebe (18, 19a, 19b) aufweist, insbesondere wobei das Schneckengetriebe (18, 19a, 19b) eine Selbsthemmung aufweist.

6. Tibiales Probeimplantat (1) nach Anspruch 5, **dadurch gekennzeichnet, dass** das Schneckengetriebe (18, 19a, 19b) eine Schneckenantriebswelle (18) aufweist, die um eine senkrecht zur Hochrichtung (Z) orientierte zweite Drehachse (20) drehbar an dem Unterteil (2) gelagert ist, wobei die Schneckenantriebswelle (18) mit einem verzahnten Außenumfangsabschnitt (19a, 19b) des wenigstens einen Antriebsrads (6a, 6b) oder mit einem dem wenigstens einen Antriebsrad (6a, 6b) vorgeschalteten Schneckenrad zusammenwirkt, Insbesondere wobei die Schneckenantriebswelle (18) einends einen Drehbetätigungsabschnitt (21) aufweist, der zur manuellen und/oder werkzeuggetriebenen Drehbetätigung der Schneckenantriebswelle (18) um die zweite Drehachse (20) eingerichtet ist.

7. Tibiales Probeimplantat (1) nach einem der vorhergehenden Ansprüche, **dadurch gekennzeichnet, dass** das wenigstens eine Antriebsrad (6a, 6b) und/oder die Schneckenantriebswelle (18) an einer Gleitlagerfläche (25b) des Unterteils (2) gleitend drehbar gelagert ist, wobei die Gleitlagerfläche (25b) mehrere radiale Spülausbuchtungen (26b) aufweist.

8. Tibiales Probeimplantat (1, 100) nach einem der vorhergehenden Ansprüche, **dadurch gekennzeichnet, dass** das Unterteil (2, 102) zweischalig gestaltet ist und eine Oberschale (2a, 102a) und eine Unterschale (2b, 102b) aufweist, wobei das wenigstens eine Antriebsrad (6a, 6b, 106a, 106b) und/oder die Schneckenantriebswelle (18) zwischen der Oberschale (2a, 102a) und der Unterschale (2b, 102b) gehalten ist.

9. Tibiales Probeimplantat (1, 100) nach einem der vorhergehenden Ansprüche, **dadurch gekennzeichnet, dass** der Höheneinstellmechanismus (E, E') in Bezug auf eine vertikale Mittellängsebene (X-Z) wenigstens weitgehend, vorzugsweise vollständig, spiegelsymmetrisch gestaltet ist, wobei wenigstens zwei Antriebsräder (6a, 6b, 106a, 106b) und wenigstens zwei Abtriebselemente (7a, 7b, 107a, 107b) vorgesehen sind.

10. Tibiales Probeimplantat (100) nach einem der vorhergehenden Ansprüche, **dadurch gekennzeichnet, dass** wenigstens zwei Antriebsräder (106a, 106b) und ein Steuerrad (118) vorhanden sind, die jeweils eine Stirnverzahnung (119a, 119b, 119) aufweisen, wobei das Steuerrad (118) mit beiden Antriebsrädern (106a, 106b) stirnverzahnt ist.

11. Tibiales Probeimplantat (100) nach Anspruch 10, **dadurch gekennzeichnet, dass** das wenigstens eine Steuerrad (118) eine weitere Steuerkurve (109c) aufweist, die mit einem weiteren Stützabschnitt (110c) eines weiteren Abtriebselements (107c) zusammenwirkt.

12. Tibiales Probeimplantat (100) nach einem der vorhergehenden Ansprüche, **dadurch gekennzeichnet, dass** wenigstens ein drehfest mit dem wenigstens einen Antriebsrad (106a, 106b) verbundenes Mitnehmerelement (132a, 132b) vorhanden ist, das intermittierend mit dem Anzeigerad (130a, 130b) zusammenwirkt, wodurch eine kontinuierliche Drehbewegung des Antriebsrads (106a, 106b) eine schrittweise Drehbewegung des Anzeigerads (130a, 130b) bewirkt.

13. Tibiales Probeimplantat (100) nach einem der vorhergehenden Ansprüche, **dadurch gekennzeichnet, dass** eine Rasteinrichtung vorhanden ist, die ein drehfest mit dem wenigstens einen Antriebsrad (106a, 106b) wirkverbundenes Rastelement (135) und ein an dem Unterteil (102) angeordnetes komplementäres Gegenrastelement (139) aufweist, wobei das Rastelement (135) und das Gegenrastelement (139) in definierten Drehstellungen des wenigstens einen Antriebsrads (106a, 106b) unter Ausbildung einer überrastbaren Rastverbindung zusammenwirken.

14. Tibiales Probeimplantat (100) nach einem der vorhergehenden Ansprüche, **dadurch gekennzeichnet, dass** ein mit dem Unterteil (102) lösbar verbindbarer Handgriff (G) mit einer Betätigungsmechanik (B) vorhanden ist, wobei die Betätigungsmechanik (B) ein Betätigungsrad (154) aufweist, das in einem mit dem Unterteil (102) verbundenen Zustand des Handgriffs (G) mit dem wenigstens einen Antriebsrad (106a, 106b) wirkverbunden ist.

15. Tibiales Probeimplantat (100) nach Anspruch 14, **dadurch gekennzeichnet, dass** der Handgriff (G) wenigstens ein an seinem distalen Ende (151) angeordnetes Verbindungselement (152) aufweist, das zur Verbindung des Handgriffs (G) mit dem Unterteil (102) lösbar mit einem komplementären Verbindungselement (153) des Unterteils (102) verbindbar ist.

## Claims

1. Tibial trial implant (1, 100) for use in a knee joint replacement operation, having
- a lower part (2, 102), which is provided for tibial fixation,
- an upper part (3, 103) arranged above the lower part (2, 102) in the height direction (Z) and having a glide face (5, 105) which is arranged on an upper side and which is provided for gliding interaction with a femoral component,
- a height adjustment mechanism (E, E'), which is operatively connected to the upper part (3, 103) and the lower part (2, 102) and by means of which the upper part (3, 103) can be guided displaceably relative to the lower part (2, 102) in the height direction (Z) between
- a first adjustment position, in which the glide face (5, 105) is positioned at a first height (H1) above the lower part (2, 102), and
- a second adjustment position, in which the glide face (5, 105) is positioned at a second height (H2) above the lower part (2, 102),
- wherein the height adjustment mechanism (E, E') has a cam gear with at least one drive wheel (6a, 6b, 106a, 106b), which is mounted rotatably on the lower part (2, 102) about a first rotation axis (8a, 8b) oriented parallel to the height direction (Z) and has a control cam (9a, 9b, 109a, 109b) rising in the height direction (Z), and with at least one driven element (7a, 7b, 107a, 107b), which is firmly connected to the upper part (3, 103) and has a supporting section (10a, 10b, 110a, 110b) glidingly supported on the control cam (9a, 9b, 109a, 109b), as a result of which the upper part (3, 103) can be displaced, by means of a rotational movement of the drive wheel (6a, 6b, 106a, 106b), between the first adjustment position and the second adjustment position,
- **characterized in that** a scale display (S) is formed between the lower part (2) and the at least one drive wheel (6a, 6b), which scale display (S) has a reading element and a scale and allows reading of the adjustment position of the upper part (3) and/or of the height (H1, H2) of the glide face (5), or
- **in that** the at least one drive wheel (106a, 106b) is coupled so as to transmit movement to at least one display wheel (130a, 130b) which is rotationally mounted on the lower part (102), the display wheel (130a, 130b) having a scale (S') which allows reading of the adjustment position of the upper part (103) and/or of the height of the glide face (105).

2. Tibial trial implant (1, 100) according to Claim 1, **characterized in that** the control cam (9a, 109a) is formed by a control face (11a, 111a) helically extending with a constant pitch coaxially around the first rotation axis (8a).

3. Tibial trial implant (1, 100) according to Claim 1 or 2, **characterized in that** the supporting section (10a, 110a) is formed by a supporting face (12a, 112a) helically extending with a constant pitch coaxially around the first rotation axis (8a, 108a).

4. Tibial trial implant (1) according to one of the preceding claims, **characterized in that** the upper part (3) and the lower part (2) are releasably plugged together by means of a plug connection formed between the at least one drive wheel (6a) and the at least one driven element (7a) while being glidingly movable relative to each other along a plug axis (17a), the plug axis (17a) being oriented coaxially to the first rotation axis (8a), in particular with the at least one drive wheel (6a) having a cylindrical bore (14a) which extends coaxially to the first rotation axis (8a) and into which a complementary plug cylinder (16a) of the at least one driven element (7a) is releasably plugged so as to form the plug connection.

5. Tibial trial implant (1) according to one of the preceding claims, **characterized in that** the height adjustment mechanism (E) has a worm gear (18, 19a, 19b) preceding the cam gear, in particular with the worm gear (18, 19a, 19b) having self-retention.

6. Tibial trial implant (1) according to Claim 5, **characterized in that** the worm gear (18, 19a, 19b) has a worm drive shaft (18), which is mounted rotatably on the lower part (2) about a second rotation axis (20) oriented perpendicularly to the height direction (Z), the worm drive shaft (18) interacting with a toothed outer circumferential section (19a, 19b) of the at least one drive wheel (6a, 6b) or with a worm wheel preceding the at least one drive wheel (6a, 6b), in particular with the worm drive shaft (18) having at one end a rotation actuation section (21), which is configured for manual and/or tool-driven rotation actuation of the worm drive shaft (18) about the second rotation axis (20).

7. Tibial trial implant (1) according to one of the preceding claims, **characterized in that** the at least one drive wheel (6a, 6b) and/or the worm drive shaft (18) is glidingly mounted rotatably on a glide bearing face (25b) of the lower part (2), the glide bearing face (25b) having a plurality of radial flushing protrusions (26b).

8. Tibial trial implant (1, 100) according to one of the preceding claims, **characterized in that** the lower part (2, 102) is configured as two shells and has an upper shell (2a, 102a) and a lower shell (2b, 102b), the at least one drive wheel (6a, 6b, 106a, 106b) and/or the worm drive shaft (18) being held between the upper shell (2a, 102a) and the lower shell (2b, 102b).

9. Tibial trial implant (1, 100) according to one of the preceding claims, **characterized in that** the height adjustment mechanism (E, E') is configured at least substantially, preferably fully, mirror-symmetrically with respect to a vertical mid-length plane (X-Z), at least two drive wheels (6a, 6b, 106a, 106b) and at least two driven elements (7a, 7b, 107a, 107b) being provided.

10. Tibial trial implant (100) according to one of the preceding claims, **characterized in that** there are provided at least two drive wheels (106a, 106b) and one control wheel (118), which each have a spur toothing (119a, 119b, 119), the control wheel (118) being spurmeshed with the two drive wheels (106a, 106b).

11. Tibial trial implant (100) according to Claim 10, **characterized in that** the at least one control wheel (118) has a further control cam (109c), which interacts with a further supporting section (110c) of a further driven element (107c).

12. Tibial trial implant (100) according to one of the preceding claims, **characterized in that** there is provided at least one driver element (132a, 132b) which is connected for conjoint rotation to the at least one drive wheel (106a, 106b) and which intermittently interacts with the display wheel (130a, 130b), as a result of which a continuous rotational movement of the drive wheel (106a, 106b) causes a stepwise rotational movement of the display wheel (130a, 130b).

13. Tibial trial implant (100) according to one of the preceding claims, **characterized in that** there is provided a latch device which has a latch element (135), operatively connected to the at least one drive wheel (106a, 106b) for conjoint rotation, and a complementary counterpart latch element (139) arranged on the lower part (102), the latch element (135) and the counterpart latch element (139) interacting in defined rotational settings of the at least one drive wheel (106a, 106b) while forming an overridable latch connection.

14. Tibial trial implant (100) according to one of the preceding claims, **characterized in that** there is provided a handle (G) which is releasably connectable to the lower part (102) and has an actuation mechanism (B), the actuation mechanism (B) having an actuation wheel (154) which is operatively connected to the at least one drive wheel (106a, 106b) in a state when the handle (G) is connected to the lower part (102).

15. Tibial trial implant (100) according to Claim 14, **characterized in that** the handle (G) has at least one connection element (152) which is arranged at its distal end (151) and which is releasably connectable to a complementary connection element (153) of the lower part (102) in order to connect the handle (G) to the lower part (102).

## Revendications

1. Implant d'essai tibial (1, 100) destiné à être utilisé en chirurgie de remplacement de l'articulation du genou, ledit implant comprenant
- une partie inférieure (2, 102) destinée à la fixation au tibia,
- une partie supérieure (3, 103) qui est disposée au-dessus de la partie inférieure (2, 102) dans la direction verticale (Z), qui est pourvue d'une surface de glissement (5, 105) disposée du côté supérieur et qui est destinée à coopérer de manière coulissante avec un composant fémoral,
- un mécanisme de réglage de hauteur (E, E') qui est relié fonctionnellement à la partie supérieure (3, 103) et à la partie inférieure (2, 102) qui permet de déplacer la partie supérieure (3, 103) par rapport à la partie inférieure (2, 102) de manière guidée dans la direction verticale (Z) entre
- une première position de réglage dans laquelle la surface de glissement (5, 105) est positionnée à une première hauteur (H1) au-dessus de la partie inférieure (2, 102), et
- une deuxième position de réglage dans laquelle la surface de glissement (5, 105) est positionnée à une deuxième hauteur (H2) au-dessus de la partie inférieure (2, 102),
- le mécanisme de réglage de hauteur (E, E') comportant une transmission à came qui comprend au moins une roue d'entraînement (6a, 6b, 106a, 106b), laquelle est montée sur la partie inférieure (2, 202) de manière à pouvoir tourner sur un premier axe de rotation (8a, 8b) orienté parallèlement à la direction verticale (Z) et comporte une came de commande (9a, 9b, 109a, 109b) montant dans la direction verticale (Z), et qui comprend au moins un élément de sortie (7a, 7b, 107a, 107b), lequel est relié de manière fixe à la partie supérieure (3, 103) et comporte une portion d'appui (10a, 10b, 110a, 110b) qui est en appui de manière glissante sur la came de commande (9a, 9b, 109a, 109b), ce qui permet de déplacer la partie supérieure (3, 103) entre la première position de réglage et la deuxième position de réglage par un mouvement de rotation de la roue d'entraînement (6a, 6b, 106a, 106b),
**caractérisé en ce que**
- un affichage gradué (S), qui comporte un élément de lecture et une échelle graduée et qui permet une lecture de la position de réglage de la partie supérieure (3) et/ou la hauteur (H1, H2) de la surface de glissement (5), est formé entre la partie inférieure (2) et l'au moins une roue d'entraînement (6a, 6b) ou
- l'au moins une roue d'entraînement (106a, 106b) est accouplée en transmission de mouvement à au moins une roue d'affichage (130a, 130b) montée de manière rotative sur la partie inférieure (102), la roue d'affichage (130a, 130b) comportant une échelle graduée (S') qui permet de lire la position de réglage de la partie supérieure (103) et/ou la hauteur de la surface de glissement (105).

2. Implant d'essai tibial (1, 100) selon la revendication 1, **caractérisé en ce que** la came de commande (9a, 109a) est formée par une surface de commande (11a, 111a) qui s'étend coaxialement en hélice avec un pas constant autour du premier axe de rotation (8a) .

3. Implant d'essai tibial (1, 100) selon la revendication 1 ou 2, **caractérisé en ce que** la portion d'appui (10a, 110a) est formée par une surface d'appui (12a, 112a) qui s'étend coaxialement en hélice avec un pas constant autour du premier axe de rotation (8a, 108a).

4. Implant d'essai tibial (1) selon l'une des revendications précédentes, **caractérisé en ce que** la partie supérieure (3) et la partie inférieure (2) sont enfichées conjointement de manière amovible et de façon à pouvoir effectuer un mouvement de glissement l'une par rapport à l'autre le long de l'axe d'enfichage (17a) au moyen d'une liaison d'enfichage, formée entre l'au moins une roue d'entraînement (6a) et l'au moins un élément de sortie (7a), l'axe d'enfichage (17a) étant orienté coaxialement au premier axe de rotation (8a), en particulier l'au moins une roue d'entraînement (6a) comportant un alésage de cylindre (14a) qui s'étend coaxialement au premier axe de rotation (8a) et dans lequel un cylindre d'enfichage complémentaire (16a) de l'au moins un élément de sortie (7a) est inséré de manière amovible pour former la liaison d'enfichage.

5. Implant d'essai tibial (1) selon l'une des revendications précédentes, **caractérisé en ce que** le mécanisme de réglage de hauteur (E) comporte une transmission à vis sans fin (18, 19a, 19b) montée en amont de la transmission à came, notamment la transmission à vis sans fin (18, 19a, 19b) comportant un mécanisme autobloquant.

6. Implant d'essai tibial (1) selon la revendication 5, **caractérisé en ce que** la transmission à vis sans fin (18, 19a, 19b) comporte un arbre d'entraînement de vis sans fin (18) qui est monté sur la partie inférieure (2) de manière à pouvoir tourner sur un deuxième axe de rotation (20) orienté perpendiculairement à la direction verticale (Z), l'arbre d'entraînement de vis sans fin (18) coopérant avec une portion périphérique extérieure dentée (19a, 19b) de l'au moins une roue d'entraînement (6a, 6b) ou avec une roue à vis sans fin montée en amont de l'au moins une roue d'entraînement (6a, 6b), en particulier l'arbre d'entraînement de vis sans fin (18) comportant à une extrémité une portion d'actionnement en rotation (21) qui est conçue pour actionner en rotation, manuellement et/ou par le biais d'un outil, l'arbre d'entraînement de vis sans fin (18) sur le deuxième axe de rotation (20).

7. Implant d'essai tibial (1) selon l'une des revendications précédentes, **caractérisé en ce que** l'au moins une roue d'entraînement (6a, 6b) et/ou l'arbre d'entraînement de vis sans fin (18) sont montés de manière rotative et de façon coulissante sur une surface de palier lisse (25b) de la partie inférieure (2), la surface de palier lisse (25b) comportant une pluralité d'indentations de rinçage radiales (26b).

8. Implant d'essai tibial (1, 100) selon l'une des revendications précédentes, **caractérisé en ce que** la partie inférieure (2, 102) est conçue avec deux coques et comporte une coque supérieure (2a, 102a) et une coque inférieure (2b, 102b), l'au moins une roue d'entraînement (6a, 6b, 106a, 106b) et/ou l'arbre d'entraînement de vis sans fin (18) étant maintenus entre la coque supérieure (2a, 102a) et la coque inférieure (2b, 102b).

9. Implant d'essai tibial (1, 100) selon l'une des revendications précédentes, **caractérisé en ce que** le mécanisme de réglage de hauteur (E, E') est conçu pour être au moins dans un large mesure, de préférence totalement, à symétrique de miroir par rapport à un plan longitudinal central vertical (X-Z), au moins deux roues d'entraînement (6a, 6b, 106a, 106b) et au moins deux éléments de sortie (7a, 7b, 107a, 107b) étant prévus.

10. Implant d'essai tibial (100) selon l'une des revendications précédentes, **caractérisé en ce qu'**au moins deux roues d'entraînement (106a, 106b) et une roue de commande (118) sont présente qui comportent chacune une denture frontale (119a, 119b, 119), la roue de commande (118) comportant une denture frontale s'engrenant avec les deux roues d'entraînement (106a, 106b).

11. Implant d'essai tibial (100) selon la revendication 10, **caractérisé en ce que** l'au moins une roue de commande (118) comporte une autre came de commande (109c) qui coopère avec une autre portion d'appui (110c) d'un autre élément de sortie (107c).

12. Implant d'essai tibial (100) selon l'une des revendications précédentes, **caractérisé en ce qu'**au moins un élément d'entraînement (132a, 132b), relié solidairement en rotation à l'au moins une roue d'entraînement (106a, 106b), est présent qui coopère par intermittence avec la roue d'affichage (130a, 130b), ce qui permet à un mouvement de rotation continu de la roue d'entraînement (106a, 106b) de générer un mouvement de rotation progressif de la roue d'affichage (130a, 130b).

13. Implant d'essai tibial (100) selon l'une des revendications précédentes, **caractérisé en ce qu'**un dispositif d'encliquetage est présent qui comporte un élément d'encliquetage (135) relié fonctionnellement et solidairement en rotation à au moins une roue d'entraînement (106a, 106b) et un élément d'encliquetage homologue complémentaire (139) disposé sur la partie inférieure (102),
l'élément d'encliquetage (135) et l'élément d'encliquetage homologue (139) coopérant dans des positions de rotation définies de l'au moins une roue d'entraînement (106a, 106b) pour former une liaison à sur-encliquetage.

14. Implant d'essai tibial (100) selon l'une des revendications précédentes, **caractérisé en ce qu'**une poignée (G), pouvant être reliée de manière amovible à la partie inférieure (102) et comportant un mécanisme d'actionnement (B) est présente, le mécanisme d'actionnement (B) comportant une roue d'actionnement (154) qui est reliée fonctionnellement à l'au moins une roue d'entraînement (106a, 106b) lorsque la poignée (G) est reliée à la partie inférieure (102).

15. Implant d'essai tibial (100) selon la revendication 14, **caractérisé en ce que** la poignée (G) comporte au moins un élément de liaison (152) qui est disposé à l'extrémité distale (151) de celle-ci et qui peut être relié de manière amovible à un élément de liaison complémentaire (153) de la partie inférieure (102) afin de relier la poignée (G) à la partie inférieure (102).
